# EUROPEAN PATENT SPECIFICATION

(11) **EP 2 508 200 B1**
(45) Date of publication and mention of the grant of the patent: **06.12.2017**
(21) Application number: 10824620.8
(22) Date of filing: 07.10.2010
(51) Int. Cl.: A61K 39/00, A61K 35/12, A61K 35/28, A61K 38/51, A61P 1/04, A61P 37/06, A61P 43/00, C12N 5/0783, C12N 9/88, C12N 15/09

(54) **CARBONIC ANHYDRASE I SERVING AS NOVEL ANTIGEN TO BE USED FOR TREATMENT OF AUTOIMMUNE DISEASES**
CARBOANHYDRASE I ALS NEUES ANTIGEN ZUR BEHANDLUNG VON AUTOIMMUNERKRANKUNGEN
ANHYDRASE CARBONIQUE I SERVANT DE NOUVEL ANTIGÈNE À UTILISER POUR LE TRAITEMENT DE MALADIES AUTOIMMUNES

(30) Priority: 21.10.2009 JP 2009242491
(43) Date of publication of application: 10.10.2012
(73) Proprietor: National University Corporation Ehime University, Ehime 7908577 (JP)
(72) Inventor: MURAKAMI, Hidehiro, Uwajima-shi Ehime 7988510 (JP); YAMANISHI, Hirofumi, Toon-shi Ehime 791-0295 (JP); ONJI, Morikazu, Toon-shi Ehime 791-0295 (JP)
(74) Representative: J A Kemp
(86) International application number: PCT/JP2010/006018
(87) International publication number: WO 2011/048766

(56) References cited:
- WO-A1-99/56763
- PEDERSEN, A.E. ET AL.: "Tolerogenic dendritic cells pulsed with enterobacterial extract suppress development of colitis in the severe combined immunodeficiency transfer model", IMMUNOLOGY, vol. 121, August 2007 (2007-08), pages 526-532, XP002698276,
- CONG, Y. ET AL.: 'CD4+ T cells reactive to enteric bacterial antigens in spontaneously colitic C3H/HeJBir mice: increased T helper cell type 1 response and ability to transfer disease' J EXP MED vol. 187, no. 6, 16 March 1998, pages 855 - 864, XP008155019
- BRIMNES, J. ET AL.: 'Enteric bacterial antigens activate CD4(+) T cells from scid mice with inflammatory bowel disease' EUR J IMMUNOL vol. 31, no. 1, 2001, pages 23 - 31, XP008155023
- ELSON, C.O. ET AL.: 'Monoclonal anti-interleukin 23 reverses active colitis in a T cell-mediated model in mice' GASTROENTEROLOGY vol. 132, no. 7, 2007, pages 2359 - 2370, XP008155015
- ISAO NISHIMORI: 'Expression and function ot carbonic anhydrases in the pancreas' THE JOURNAL OF JAPAN PANCREAS SOCIETY vol. 22, no. 5, 2007, pages 534 - 546
- KATSUMI YOSHIDA: 'Sukagetsu Mae no Heikinka sareta Kojosen Kino o Han'ei suru Sekkekkyu Tansan Dassui Koso 1 Isozyme Oyobi Aen Nodo ni Kansuru Kenkyu' TOHOKU IGAKU ZASSHI vol. 109, no. 1, 1997, pages 49 - 52, XP008155583
- KATSUMI YOSHIDA: 'Erythrocyte Carbonic Anhydrase I and Zinc Concentrations in Thyrotoxicosis Reflect Integrated Thyroid Hormone Levels Over the Previous Few Months' THE JAPANESE JOURNAL OF CLINICAL PATHOLOGY vol. 54, 2006, page 111, XP008155024
- BARTOLOME, M.J. ET AL.: 'Low-avidity antibodies to carbonic anhydrase-I and -II in autoimmune chronic pancreatitis' SCIENTIFICWORLDJOURNAL vol. 2, 2002, pages 1560 - 1568, XP008155034
- FONTI R. ET AL: "Carbonic anhydrase I reduction in colonic mucosa of patients with active ulcerative colitis", DIG DIS SCI. 1998 SEP;43(9):2086-92., vol. 43, no. 9, 1998, pages 2086-2092,

### SEQUENCE LISTING

<110> Ehime University
<120> Tolerogenicity enhancing antigen for treatment of inflammatory bowel diseases
<130> PW10016EH
<150> JP2009-242491
   <151> 2009-10-21
<160> 9
<170> PatentIn version 3.5
<210> 1
   <211> 786
   <212> DNA
   <213> Mus musculus
<220>
   <221> CDS
   <222> (1)..(786)
<400> 1
   atg gca agt gca gac tgg gga tat gga agc gaa aat ggt cct gac caa      48
   Met Ala Ser Ala Asp Trp Gly Tyr Gly Ser Glu Asn Gly Pro Asp Gln
   1               5                   10                  15
   tgg agc aag ctg tat ccc att gcc aat gga aac aac cag tct cct att      96
   Trp Ser Lys Leu Tyr Pro Ile Ala Asn Gly Asn Asn Gln Ser Pro Ile
               20                  25                  30
   gat att aaa acc agt gaa gcc aat cat gac tcc tct ctg aaa cca ctc     144
   Asp Ile Lys Thr Ser Glu Ala Asn His Asp Ser Ser Leu Lys Pro Leu
           35                  40                  45
   agc atc tcc tat aat cct gca act gcc aaa gaa att gtt aac gtg gga     192
   Ser Ile Ser Tyr Asn Pro Ala Thr Ala Lys Glu Ile Val Asn Val Gly
       50                  55                  60
   cat tct ttc cat gta att ttt gat gac agt agc aac caa tct gtt ctg     240
   His Ser Phe His Val Ile Phe Asp Asp Ser Ser Asn Gln Ser Val Leu
   65                  70                  75                  80
   aaa ggt ggc cct ctt gct gat agc tat cgg ctc act cag ttc cat ttt     288
   Lys Gly Gly Pro Leu Ala Asp Ser Tyr Arg Leu Thr Gln Phe His Phe
                   85                  90                  95
   cac tgg ggc aac tca aac gac cat ggc tct gag cac acc gtg gat gga     336
   His Trp Gly Asn Ser Asn Asp His Gly Ser Glu His Thr Val Asp Gly
               100                 105                 110
   act aga tat tct gga gag ctt cac tta gtt cac tgg aat tct gca aag     384
   Thr Arg Tyr Ser Gly Glu Leu His Leu Val His Trp Asn Ser Ala Lys
           115                 120                 125
   tac tcc agt gct tct gaa gcc atc tcc aag gct gat ggc ctg gca atc     432
   Tyr Ser Ser Ala Ser Glu Ala Ile Ser Lys Ala Asp Gly Leu Ala Ile
       130                 135                 140
   ctt ggc gtt ttg atg aag gtt ggt cca gcc aac cca agc ctg cag aaa     480
   Leu Gly Val Leu Met Lys Val Gly Pro Ala Asn Pro Ser Leu Gln Lys
   145                 150                 155                 160
   gta ctt gat gct cta aac tca gtt aaa act aag gga aaa cga gcc cca         528
   Val Leu Asp Ala Leu Asn Ser Val Lys Thr Lys Gly Lys Arg Ala Pro
                   165                 170                 175
   ttc aca aat ttt gac cca tcc agt ctg ctt cct tca tct ctg gat tac         576
   Phe Thr Asn Phe Asp Pro Ser Ser Leu Leu Pro Ser Ser Leu Asp Tyr
               180                 185                 190
   tgg acc tac ttt ggc tct ctg act cac cct cct ctt cat gaa agt gtg         624
   Trp Thr Tyr Phe Gly Ser Leu Thr His Pro Pro Leu His Glu Ser Val
           195                 200                 205
   acc tgg gtg atc tgc aag gat agc atc agt cta agc cca gag cag ctg         672
   Thr Trp Val Ile Cys Lys Asp Ser Ile Ser Leu Ser Pro Glu Gln Leu
       210                 215                 220
   gcc cag ctc cgt ggt ctt ctg tca agt gca gag gga gag cct gca gtt         720
   Ala Gln Leu Arg Gly Leu Leu Ser Ser Ala Glu Gly Glu Pro Ala Val
   225                 230                 235                 240
   cca gtt ctg agc aac cac cgt cca ccc caa ccc ctg aag ggc aga aca         768
   Pro Val Leu Ser Asn His Arg Pro Pro Gln Pro Leu Lys Gly Arg Thr
                   245                 250                 255
   gtc aga gcc tca ttt tga                                                 786
   Val Arg Ala Ser Phe
               260
<210> 2
   <211> 261
   <212> PRT
   <213> Mus musculus
<400> 2
   Met Ala Ser Ala Asp Trp Gly Tyr Gly Ser Glu Asn Gly Pro Asp Gln
   1               5                   10                  15
   Trp Ser Lys Leu Tyr Pro Ile Ala Asn Gly Asn Asn Gln Ser Pro Ile
               20                  25                  30
   Asp Ile Lys Thr Ser Glu Ala Asn His Asp Ser Ser Leu Lys Pro Leu
           35                  40                  45
   Ser Ile Ser Tyr Asn Pro Ala Thr Ala Lys Glu Ile Val Asn Val Gly
       50                  55                  60
   His Ser Phe His Val Ile Phe Asp Asp Ser Ser Asn Gln Ser Val Leu
   65                  70                  75                  80
   Lys Gly Gly Pro Leu Ala Asp Ser Tyr Arg Leu Thr Gln Phe His Phe
                   85                  90                  95
   His Trp Gly Asn Ser Asn Asp His Gly Ser Glu His Thr Val Asp Gly
               100                 105                 110
   Thr Arg Tyr Ser Gly Glu Leu His Leu Val His Trp Asn Ser Ala Lys
           115                 120                 125
   Tyr Ser Ser Ala Ser Glu Ala Ile Ser Lys Ala Asp Gly Leu Ala Ile
       130                 135                 140
   Leu Gly Val Leu Met Lys Val Gly Pro Ala Asn Pro Ser Leu Gln Lys
   145                 150                 155                 160
   Val Leu Asp Ala Leu Asn Ser Val Lys Thr Lys Gly Lys Arg Ala Pro
                   165                 170                 175
   Phe Thr Asn Phe Asp Pro Ser Ser Leu Leu Pro Ser Ser Leu Asp Tyr
               180                 185                 190
   Trp Thr Tyr Phe Gly Ser Leu Thr His Pro Pro Leu His Glu Ser Val
           195                 200                 205
   Thr Trp Val Ile Cys Lys Asp Ser Ile Ser Leu Ser Pro Glu Gln Leu
       210                 215                 220
   Ala Gln Leu Arg Gly Leu Leu Ser Ser Ala Glu Gly Glu Pro Ala Val
   225                 230                 235                 240
   Pro Val Leu Ser Asn His Arg Pro Pro Gln Pro Leu Lys Gly Arg Thr
                   245                 250                 255
   Val Arg Ala Ser Phe
               260
<210> 3
   <211> 786
   <212> DNA
   <213> Homo sapiens
<220>
   <221> CDS
   <222> (1)..(786)
<400> 3
   atg gca agt cca gac tgg gga tat gat gac aaa aat ggt cct gaa caa           48
   Met Ala Ser Pro Asp Trp Gly Tyr Asp Asp Lys Asn Gly Pro Glu Gln
   1               5                   10                  15
   tgg agc aag ctg tat ccc att gcc aat gga aat aac cag tcc cct gtt           96
   Trp Ser Lys Leu Tyr Pro Ile Ala Asn Gly Asn Asn Gln Ser Pro Val
               20                  25                  30
   gat att aaa acc agt gaa acc aaa cat gac acc tct ctg aaa cct att          144
   Asp Ile Lys Thr Ser Glu Thr Lys His Asp Thr Ser Leu Lys Pro Ile
           35                  40                  45
   agt gtc tcc tac aac cca gcc aca gcc aaa gaa att atc aat gtg ggg          192
   Ser Val Ser Tyr Asn Pro Ala Thr Ala Lys Glu Ile Ile Asn Val Gly
       50                  55                  60
   cat tcc ttc cat gta aat ttt gag gac aac gat aac cga tca gtg ctg        240
   His Ser Phe His Val Asn Phe Glu Asp Asn Asp Asn Arg Ser Val Leu
   65                  70                  75                  80
   aaa ggt ggt cct ttc tct gac agc tac agg ctc ttt cag ttc cat ttt        288
   Lys Gly Gly Pro Phe Ser Asp Ser Tyr Arg Leu Phe Gln Phe His Phe
                   85                  90                  95
   cac tgg ggc agt aca aat gag cat ggt tca gaa cat aca gtg gat gga        336
   His Trp Gly Ser Thr Asn Glu His Gly Ser Glu His Thr Val Asp Gly
               100                 105                 110
   gtc aaa tat tct gcc gag ctt cac gta gct cac tgg aat tct gca aag        384
   Val Lys Tyr Ser Ala Glu Leu His Val Ala His Trp Asn Ser Ala Lys
           115                 120                 125
   tac tcc agc ctt gct gaa gct gcc tca aag gct gat ggt ttg gca gtt        432
   Tyr Ser Ser Leu Ala Glu Ala Ala Ser Lys Ala Asp Gly Leu Ala Val
       130                 135                 140
   att ggt gtt ttg atg aag gtt ggt gag gcc aac cca aag ctg cag aaa        480
   Ile Gly Val Leu Met Lys Val Gly Glu Ala Asn Pro Lys Leu Gln Lys
   145                 150                 155                 160
   gta ctt gat gcc ctc caa gca att aaa acc aag ggc aaa cga gcc cca        528
   Val Leu Asp Ala Leu Gln Ala Ile Lys Thr Lys Gly Lys Arg Ala Pro
                   165                 170                 175
   ttc aca aat ttt gac ccc tct act ctc ctt cct tca tcc ctg gat ttc        576
   Phe Thr Asn Phe Asp Pro Ser Thr Leu Leu Pro Ser Ser Leu Asp Phe
               180                 185                 190
   tgg acc tac cct ggc tct ctg act cat cct cct ctt tat gag agt gta        624
   Trp Thr Tyr Pro Gly Ser Leu Thr His Pro Pro Leu Tyr Glu Ser Val
           195                 200                 205
   act tgg atc atc tgt aag gag agc atc agt gtc agc tca gag cag ctg        672
   Thr Trp Ile Ile Cys Lys Glu Ser Ile Ser Val Ser Ser Glu Gln Leu
       210                 215                 220
   gca caa ttc cgc agc ctt cta tca aat gtt gaa ggt gat aac gct gtc        720
   Ala Gln Phe Arg Ser Leu Leu Ser Asn Val Glu Gly Asp Asn Ala Val
   225                 230                 235                 240
   ccc atg cag cac aac aac cgc cca acc caa cct ctg aag ggc aga aca        768
   Pro Met Gln His Asn Asn Arg Pro Thr Gln Pro Leu Lys Gly Arg Thr
                   245                 250                 255
   gtg aga gct tca ttt tga                                                786
   Val Arg Ala Ser Phe
               260
<210> 4
   <211> 261
   <212> PRT
   <213> Homo sapiens
<400> 4
   Met Ala Ser Pro Asp Trp Gly Tyr Asp Asp Lys Asn Gly Pro Glu Gln
   1               5                   10                  15
   Trp Ser Lys Leu Tyr Pro Ile Ala Asn Gly Asn Asn Gln Ser Pro Val
               20                  25                  30
   Asp Ile Lys Thr Ser Glu Thr Lys His Asp Thr Ser Leu Lys Pro Ile
           35                  40                  45
   Ser Val Ser Tyr Asn Pro Ala Thr Ala Lys Glu Ile Ile Asn Val Gly
       50                  55                  60
   His Ser Phe His Val Asn Phe Glu Asp Asn Asp Asn Arg Ser Val Leu
   65                  70                  75                  80
   Lys Gly Gly Pro Phe Ser Asp Ser Tyr Arg Leu Phe Gln Phe His Phe
                   85                  90                  95
   His Trp Gly Ser Thr Asn Glu His Gly Ser Glu His Thr Val Asp Gly
               100                 105                 110
   Val Lys Tyr Ser Ala Glu Leu His Val Ala His Trp Asn Ser Ala Lys
           115                 120                 125
   Tyr Ser Ser Leu Ala Glu Ala Ala Ser Lys Ala Asp Gly Leu Ala Val
       130                 135                 140
   Ile Gly Val Leu Met Lys Val Gly Glu Ala Asn Pro Lys Leu Gln Lys
   145                 150                 155                 160
   Val Leu Asp Ala Leu Gln Ala Ile Lys Thr Lys Gly Lys Arg Ala Pro
                   165                 170                 175
   Phe Thr Asn Phe Asp Pro Ser Thr Leu Leu Pro Ser Ser Leu Asp Phe
               180                 185                 190
   Trp Thr Tyr Pro Gly Ser Leu Thr His Pro Pro Leu Tyr Glu Ser Val
           195                 200                 205
   Thr Trp Ile Ile Cys Lys Glu Ser Ile Ser Val Ser Ser Glu Gln Leu
       210                 215                 220
   Ala Gln Phe Arg Ser Leu Leu Ser Asn Val Glu Gly Asp Asn Ala Val
   225                 230                 235                 240
   Pro Met Gln His Asn Asn Arg Pro Thr Gln Pro Leu Lys Gly Arg Thr
                   245                 250                 255
   Val Arg Ala Ser Phe
               260
<210> 5
   <211> 20
   <212> DNA
   <213> Artificial
<220>
   <223> cytosine-phosphorothioate-guanine oligodeoxynucleotide
<400> 5
   tccatgacgt tcctgatgct 20
<210> 6
   <211> 29
   <212> DNA
   <213> Artificial
<220>
   <223> murine CA I sense primer
<400> 6
   aataagatat catggcaagt gcagactgg 29
<210> 7
   <211> 32
   <212> DNA
   <213> Artificial
<220>
   <223> murine CA I antisense primer
<400> 7
   tgctggacta gtaaatgagg ctctgactgt tc 32
<210> 8
   <211> 21
   <212> DNA
   <213> Artificial
<220>
   <223> murine CA I sense primer
<400> 8
   atggcaagtg cagactgggg a 21
<210> 9
   <211> 32
   <212> DNA
   <213> Artificial Sequence
<220>
   <223> murine CA I antisense primer
<400> 9
   ccttgcggat cctcaaaatg aggctctgac tg 32

## Description

### [Technical Field]

The present invention relates to an antigen-specific tolerogenic antigen presentation cell for use in a method for treating inflammatory bowel disease.

### [Background Art]

An inflammatory bowel disease (IBD), including Crohn's disease and ulcerative colitis, is a disease characterized by an impaired immune system in the intestinal tract. Although the detailed pathogenesis have not been fully clarified, one of causes is supposed to be a sensitive natural or acquired immune response to commensal bacteria, various microbial products and food (see Non-Patent Documents 1 to 3.)

Immunomodulation in the microenvironment should be steadily fine-regulated to maintain local homeostasis. Such regulation is believed to be site-specific (for example, gastrointestinal environment-specific) and to be mediated by chronic exposure to microorganisms. A dendritic cell serves an important role in controlling the immunomodulation (see Non-patent Document 4). The dendritic cell is the most powerful and effective antigen presentation cell and is important in induction of an early immune response. The dendritic cell also plays a key role in producing immunological tolerance. It has been reported that the dendritic cell in situ induces antigen-specific immunological tolerance in the central and peripheral lymphoid organs (see Non-Patent Document 5). Although the immunological tolerance mechanism has not been completely understood, it has been reported that the dendritic cell promotes the differentiation of a CD4⁺CD25⁻ T cell into a CD4⁺CD25⁺Foxp3⁺ regulatory T cell to induce the immunological tolerance by T cell at the periphery (see Non-Patent Document 6).

Selective enhancement of the immunological tolerogenicity by the dendritic cell has been performed using an immature dendritic cell prepared by pharmacological inhibition of maturation or a recombinant dendritic cell expressing an immunosuppressive molecule (see Non-Patent Document 7). It has been also reported that in studies using mouse models, some types of the tolerogenic or regulatory dendritic cells antigen-specifically improve a pathological condition in a wide variety of diseases, such as an autoimmunity, allergy, transplant rejection and the like (see Non-Patent Documents 6 and 8 to 11).

Cecal bacterial antigen (hereinafter referred to as "CBA") is one of possible substances associated with a pathological condition of the inflammatory bowel disease. It has been reported that a mouse model of severe combined immunodeficiency develops severe enteritis when transplanted with CBA-responsive interleukin (IL)-17 producing CD4⁺T cells (see Non-Patent Documents 12 to 14). Although CBA is considered to be composed of some proteins, there are no reports of identification of the protein which acts as antigen specific to the inflammatory bowel disease.

Non-Patent document 15 discloses a reduction in the expression of CA-I in active ulcerative colitis.

### [Citation List]

### [Non-Patent Document]

Non-Patent Document 1:
   T.T. Macdonald et al., Science, 307: 1920-5 (2005)
Non-Patent Document 2:
   R.B. Sartor et al., Gastroenterology, 134: 577-94 (2008)
Non-Patent Document 3:
   D.C. Baumgart et al., Lancet, 369: 1627-40 (2007)
Non-Patent Document 4:
   Y. Belkaid et al., Immunity, 29: 362-71 (2008)
Non-Patent Document 5:
   R.M. Stenman et al., Annu. Rev. Immunol., 21: 685-711 (2003)
Non-Patent Document 6:
   S. Fujita et al., Blood, 110: 3793-803 (2007)
Non-Patent Document 7:
   H. Hackstein et al., Nat. Rev. Immunol., 4: 24-34 (2004)
Non-Patent Document 8:
   M. Menges et al., J. Exp. Med., 195: 15-21 (2002)
Non-Patent Document 9:
   M. Torisu et al., J. Gastroenterol., 43: 100-7 (2008)
Non-Patent Document 10:
   S. Fujita et al., J. Allergy Clin. Immunol., 121: 95-104 e7 (2008)
Non-Patent Document 11:
   K. Sato et al., Immunity, 18: 367-79 (2003)
Non-Patent Document 12:
   Y. Cong et al., J. Exp. Med., 187: 855-64 (1998)
Non-Patent Document 13:
   C.O. Elson et al., Gastroenterology, 132: 2359-70 (2007)
Non-Patent Document 14:
   J. Brimnes et al., Eur. J. Immunol., 31: 23-31 (2001)
Non-Patent Document 15:
   Fonti et al., Digestive Diseases and Sciences, 43: 2086-2092 (1998)

### [Summary of Invention]

### [Technical Problem]

The inflammatory bowel disease has not effectively treated based on the existing treatment strategies, and thus there has been a need for a novel and better treatment method.

The inventors of the present invention revealed that carbonic anhydrase I (i.e., CA I) and a CA I-pulsed regulatory dendritic cell have an antigen-specific therapeutic effect on inflammatory bowel disease in a model mouse, and thus completed the present invention. Specifically, by two-dimensional difference gel electrophoresis (2D-DIGE) and time-of-flight mass spectrometry (TOF-MS), a primary protein of CBA was analyzed and identified to be CA I. Then, the identified CA I pulsed regulatory dendritic cells were revealed to induce an antigen-specific depressant effect on enteritis in a colitis model mouse. Also, the inventors found that direct administration of CA I induced the antigen-specific depressant effect on enteritis in the colitis model mouse.

Accordingly, the present invention is directed to the following:
(1) Carbonic anhydrase I, a carbonic anhydrase I-specific tolerogenic antigen presentation cell or a carbonic anhydrase I-specific regulatory T-cell for use in a method of inducing immune tolerance and thereby treating or preventing inflammatory bowel disease.
(2) The carbonic anhydrase I for use according to (1),wherein the method is cell therapy based on a tolerogenic antigen presentation cell.
(3) The carbonic anhydrase I-specific tolerogenic antigen presentation cell for use according to (1), or carbonic anhydrase I for use according to (2), wherein the tolerogenic antigen presentation cell is a regulatory dendritic cell.
(4) CA for use according to (1), wherein the method comprises pulsing a tolerogenic antigen presentation cell *ex vivo* with CA I.
(5) CA I for use according to (4), wherein the method comprises:
   - taking the tolerogenic antigen presentation cell from a patient;
   - pulsing the tolerogenic antigen presentation cell with CA I; and
   - administering the pulsed tolerogenic antigen presentation cell into the patient.
(6) CA I for use according to (4) or (5), wherein the tolerogenic antigen presentation cell is a regulatory dendritic cell.
(7) An *ex vivo* method for producing an antigen-specific tolerogenic antigen presentation cell, comprising pulsing a tolerogenic antigen presentation cell obtained from a patient with CA I.
(8) An *ex vivo* method for producing an antigen-specific regulatory T cell, comprising:
   - preparing a tolerogenic antigen presentation cell and a naive T cell;
   - pulsing the tolerogenic antigen presentation cell with CA I; and
   - contacting the pulsed tolerogenic antigen presentation cell with the naive T cell.
(9) The method of (7) or (8), wherein the tolerogenic antigen presentation cell is a regulatory dendritic cell.
(10) An isolated CA I-specific immunogenic antigen presentation cell.
(11) The cell of (10), wherein the immunogenic antigen presentation cell is a regulatory dendritic cell.
(12) An isolated CA I-specific regulatory T cell.

The term "cell therapy" herein means a cell for use in a method of treatment of diseases, characterized by administration of the cell. The cell may be derived from a patient to be administered, or may be derived from another individual. Preferably, the cell derived from a patient to be administered is used. In general, the cell therapy comprises a step of obtaining and isolating cells, a step of treating the cells and a step of administering the treated cells into the patient. The cell therapy may further comprise a step of proliferating the cell. The phrase "a cell therapy based on a tolerogenic antigen presentation cell" means a tolerogenic antigen presentation cell for use in a method of treatment of diseases, characterized by administration of the tolerogenic antigen presentation cell.

Generally, "carbonic anhydrase I (CA I)" is a metalloenzyme which is present in cytoplasm and reversibly catalyzes a hydration reaction of carbon dioxide. As used herein, the "CA I" does not have to be a full-length CA I and may also include its partial peptide as long as it is able to induce the CA I-specific immunogenic antigen presentation cell. Further, as used in this specification, the CA I (including its partial peptide; the same shall apply hereinafter) may be modified by substituting, deleting, adding and/or inserting a part (for example, a few) of amino acid sequence as long as it is able to induce the CA I-specific immunogenic antigen presentation cell. Also, as used in this specification, the CA I may be consisted of the amino acid sequence with homology of 50 % or more, 60 % or more, 75 % or more, 85 % or more, 90 % or more, 95 % or more, 97 % or more, 98 % or more, or 99 % or more to the amino acid sequence of CA I as long as it is able to induce the CA I-specific immunogenic antigen presentation cell. Alternatively, as used herein, the CA I may be a protein encoded by a genome sequence which can hybridize with the genome sequence of CA I under a stringent condition (Molecular Cloning, A Laboratory Manual, Second Edition, Cold Spring Harbor Laboratory Press (1989)) as long as it is able to induce the CA I-specific immunogenic antigen presentation cell. The CA I may be appropriately modified to improve ability to induce immunological tolerance. The CA I may also be a fused protein with a substance which improves ability to induce immunological tolerance, or with a substance with ability to induce immunological tolerance. Preferably, the CA I may be a protein or a peptide included in above said CA I of the present specification, wherein an immunogenic antigen presentation cell induced by the protein or the peptide can produce immunological tolerance and can treat or prevent autoimmune disease (inflammatory bowel disease in the present invention).

The term "a tolerogenic antigen presentation cell" refers to the antigen presentation cell which can induce immunological tolerance. The tolerogenic antigen presentation cell may be a cell obtained from a patient which is subjected to be treated, or a cell obtained from those other than the patient, and preferably is the cell obtained from the patient. The tolerogenic antigen presentation cell may include, for example, a regulatory dendritic cell. The "regulatory dendritic cell" is not particularly limited as long as it is a subset of the dendritic cell involved in the immunological tolerance, which may be also referred to as a tolerogenic dendritic cell. The regulatory dendritic cell may encompass a dendritic cell having ability to induce immunosuppression through the production of cytokine such as interleukin 10, a dendritic cell having ability to generate a regulatory T cell (for example, a CD4⁺CD25⁺Foxp3⁺ regulatory T cell) and/or a dendritic cell having ability to control the function of the T cell. For example, the regulatory dendritic cell may include a dendritic cell without expressing costimulatory molecules, such as CD40, CD80 and CD86, on its surface; and a dendritic cell expressing CCR9 on its surface (H. Hadeiba et al., Nat. Immunol., 9(11): 1253-60 (2008)); a dendritic cell expressing miR-155 on its surface (A.W. Pedersen et al., Clin. Exp. Immunol., 157(1): 48-59 (2009)); and a dendritic cell expressing CD200 receptor 3 on its surface (K. Sato et al., Blood., 1173: 4780-9 (2009)).

The term "regulatory T cell" refers to a T cell having a role to inhibit activity of another T cell with the ability to damage tissue. The regulatory T cell may be a cell obtained from a patient who is subjected to be treated, or a cell obtained from those other than the patient, and preferably is the cell obtained from the patient. More preferably, the regulatory T cell is a T cell expressing Foxp3, CD4 and CD25 molecules on its surface.

The term "a naive T cell" refers to a T cell which has not contacted with the specific antigen, and preferably is CD4+CD25- naive T cell.

The term "immunological tolerance" means decrease in immune response level, delay in occurrence or progress of immune reaction and/or the decrease in risk from immune reaction. The term antigen-specific immunological tolerance means the immunological tolerance produced especially to a given antigen, compared with the other antigen.

As used herein, the term "an autoimmune disease" refers to a disease developed by occurrence of immune response to a self-antigen. In the present invention, the autoimmune disease is inflammatory bowel disease. The term "inflammatory bowel disease" refers to a disease characterized by chronic persistent enteritis, and includes ulcerative colitis, Crohn's disease and the like.

### [Advantageous Effects of Invention]

Since the cell therapy using CA I as the antigen according to the present invention can improve symptom in autoimmune disease, especially the symptom in inflammatory bowel disease, it is useful in a method for treating or preventing inflammatory bowel disease, which has been difficult to treat.

### [Brief Description of Drawings]

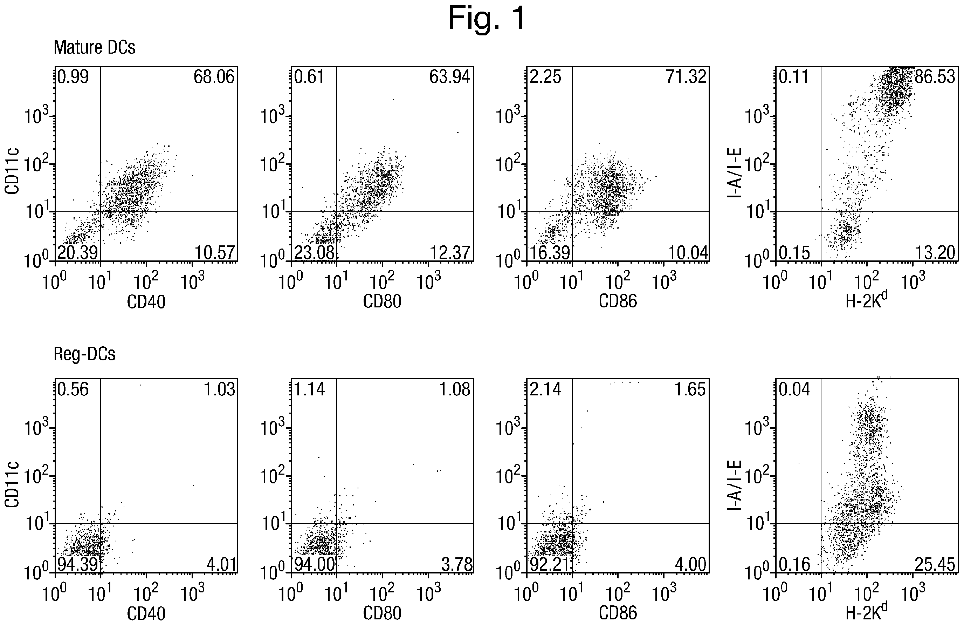

Fig.1 is a dot-plot showing results of analysis of expression of cell-surface molecules in the mature and the regulatory dendritic cells by the flow cytometry. In Fig.1, "Mature DCs" indicates data of the mature dendritic cells, and "Reg-DCs" indicates data of the regulatory dendritic cells. In the three data on the left of both of "Mature DCs" and "Reg-DCs", the vertical axis indicates an expression level of CD11c, and the horizontal axis indicates each expression levels of CD40, CD80 and CD86 in order from left to right, respectively. In the rightmost data, the vertical axis indicates an expression level of I-A/I-E, and the horizontal axis indicates an expression level of H-2K^{d}.

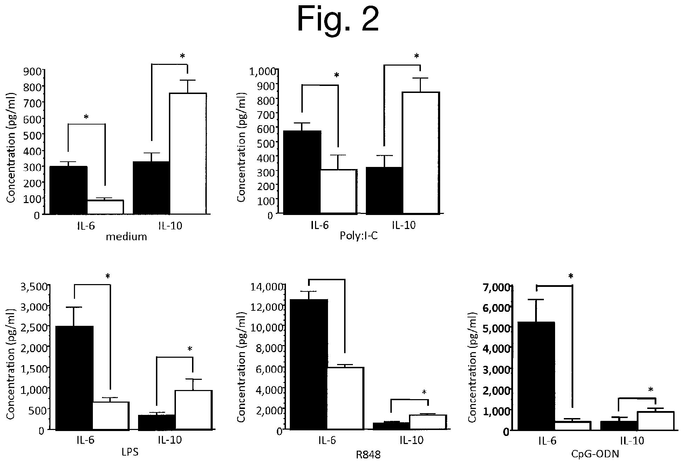

Fig. 2 is a graph showing results of measurement of production of IL-6 and IL-10 in a supernatant by a Cytometric Bead Array (BD Biosciences Pharmingen, San Diego, CA, US) after stimulation of mature dendritic cell and regulatory dendritic cell with polyinosinic-poly:I-C, ultrapure LPS, R848 or CpG-ODN for 24 hours. An error bar indicates standard deviation (hereinafter referred to as "SD"). In Fig. 2, the vertical axis indicates a level of each cytokine (pg/mL), and the horizontal axis indicates a type of measured cytokine. A single asterisk means P < 0.01 compared with the mature dendritic cell.

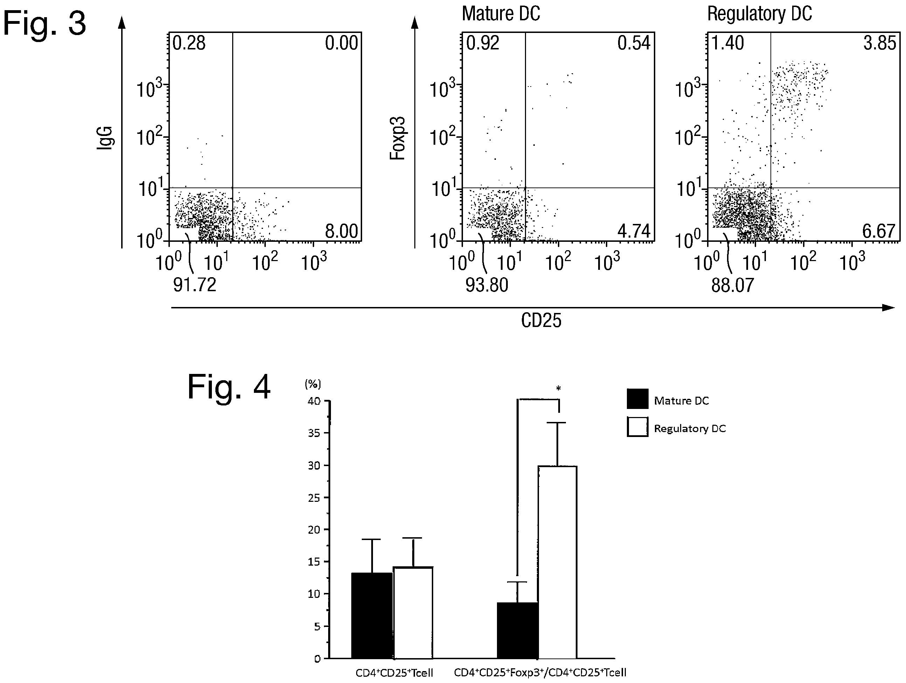

Fig. 3 is a dot-plot showing results of the flow cytometry analysis of CD4⁺CD25⁺Foxp3⁺ regulatory T cells induced in vitro by using the mature dendritic cell or the regulatory dendritic cell. The leftmost plot shows expression of an isotypic control IgG and CD25 analyzed by flow cytometry after stimulation of CD4⁺CD25⁻ T cell with regulatory dendritic cell. In the leftmost plot, the vertical axis indicates expression level of isotypic control IgG, and the horizontal axis indicates expression level of CD25. "Mature DC" shows expression of Foxp3 and CD25 analyzed by flow cytometry after simulation of CD4⁺CD25⁻ T cell with mature dendritic cells, wherein the vertical axis indicates expression level of Foxp3, and the horizontal axis indicates expression level of CD25. "Regulatory DC" shows expression of Foxp3 and CD25 analyzed by flow cytometry after stimulation of CD4⁺CD25⁻ T cells with regulatory dendritic cell, wherein the vertical axis indicates expression level of Foxp3, and the horizontal axis indicates expression level of CD25.
Fig. 4 is a graph showing a ratio of the produced T cell subset calculated from the flow cytometry data of Fig. 3. In Fig. 4, the vertical axis indicates the ratio. On the horizontal axis, "CD4⁺CD25⁺ T Cell" indicates the ratio of the produced CD4⁺CD25⁺ T cells, and "CD4⁺CD25⁺Foxp3⁺/CD4⁺CD25⁺ T cell" indicates the ratio of the produced CD4⁺CD25⁺Foxp3⁺ T cells relative to the produced CD4⁺CD25⁺ T cells. An error bar indicates SD. A single asterisk means P < 0.01 compared with the mature dendritic cell.

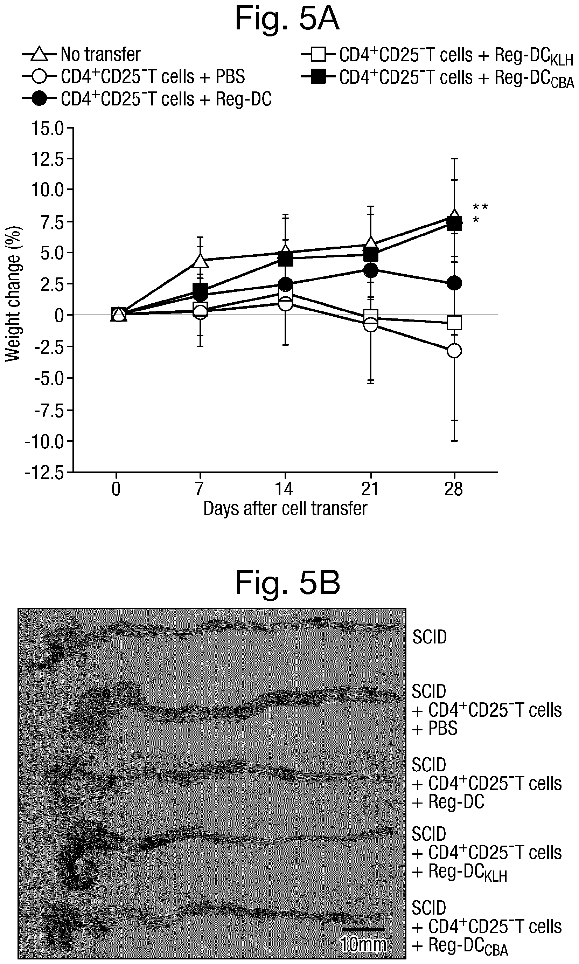

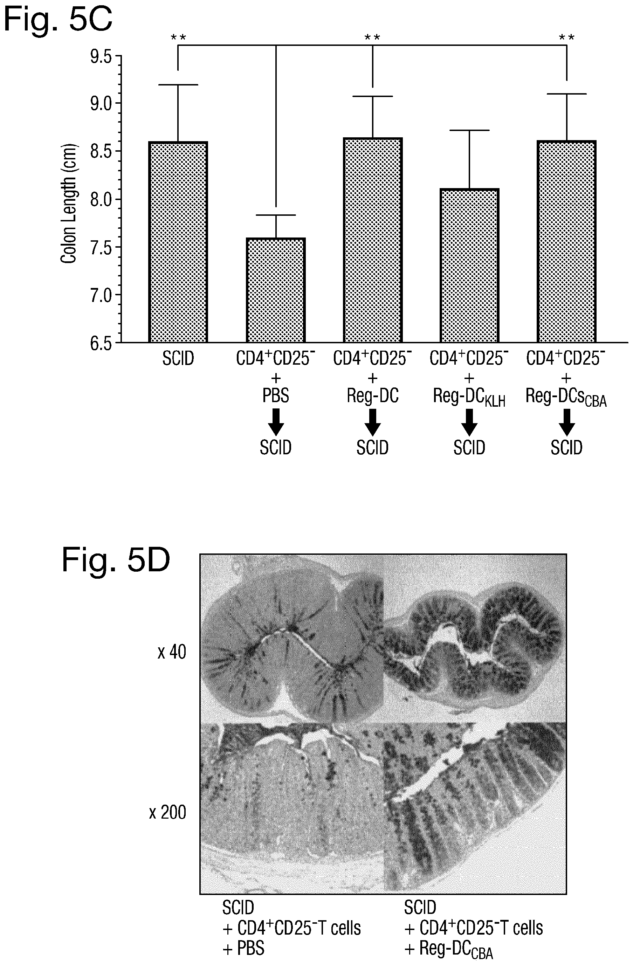

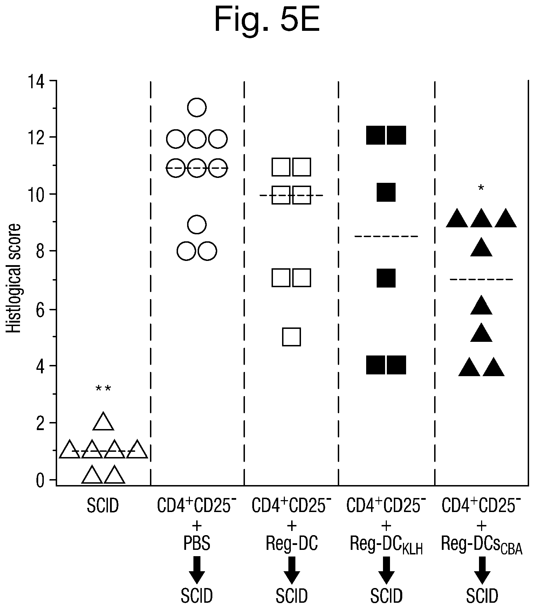

Fig. 5 shows results of experiment using a colitis model mouse induced by the CD4⁺CD25⁻ T cell. Fig. 5A is a graph of time course of change in body weight (%). In Fig. 5A, the vertical axis indicates the body weight change (%), and the horizontal axis indicates a number of day(s) after cell transplantation. In Fig. 5A, a triangle indicates a mouse without transplanted CD4⁺CD25⁻ T cell; an open circle indicates a mouse with transplanted CD4⁺CD25⁻ T cell which was administered with PBS; a filled circle indicates a mouse with transplanted CD4⁺CD25⁻ T cell which was administered with the antigen-nonpulsed regulatory dendritic cell; an open square indicates a mouse with transplanted CD4⁺CD25⁻ T cell which was administered with the KLH-pulsed regulatory dendritic cell; and a filled square indicates a mouse with transplanted CD4⁺CD25⁻ T cell which was administered with the CBA-pulsed regulatory dendritic cell. An error bar indicates SD. Fig. 5B is a photograph of macroscopic findings of colon at 28 days after the cell transplantation of the CD4⁺CD25⁻ T cell. In the photograph, in descending order, "SCID" indicates a mouse without transplanted CD4⁺CD25⁻ T cell; "SCID + CD4⁺CD25⁻ T cells + PBS" indicates a mouse with transplanted CD4⁺CD25⁻ T cell which was administered with PBS; "SCID + CD4⁺CD25⁻ T cells + Reg-DC" indicates a mouse with transplanted CD4⁺CD25⁻ T cell which was administered the antigen-nonpulsed regulatory dendritic cell; "SCID + CD4⁺CD25⁻ T cells + Reg-DC_{KLH}" indicates a mouse with transplanted CD4⁺CD25⁻ T cell which was administered with the KLH-pulsed regulatory dendritic cell; and "SCID + CD4⁺CD25⁻ T cells + Reg-DC_{CBA}" indicates a mouse with transplanted CD4⁺CD25⁻ T cell which was administered with the CBA-pulsed regulatory dendritic cell. Scale bar on the bottom-right equals 10 mm. Fig. 5C is a graph of measured length of the colon shown in Fig. 5B. In Fig. 5C, the vertical axis indicates the length of the colon in cm, and the horizontal axis indicates the mouse used for the colon length measurement. An error bar indicates SD. Fig. 5D is a photograph of hematoxylin-eosin (H&E) stained colon at 28 days after cell transplantation of CD4⁺CD25⁻ T cell. The upper photographs of Fig. 5D is enlarged images at a magnification of 40 times, and the lower photographs of Fig. 5D is enlarged images at a magnification of 200 times. In Fig. 5D, the left images show colon tissues of a mouse with transplanted CD4⁺CD25⁻ T cell which was administered with PBS, and the right images show colon tissues of a mouse with transplanted CD4⁺CD25⁻ T cell which was administered with CBA-pulsed regulatory dendritic cells. Fig. 5E is a graph showing calculated histological scores. In Fig. 5E, the vertical axis indicates a histological score, and the horizontal axis indicates the mouse used for the histological scoring. A horizontal line therein indicates a median value. A single asterisk means P < 0.05 compared with the mouse which was administered with PBS, and a double asterisk means P < 0.01 compared with the mouse which was administered with PBS.

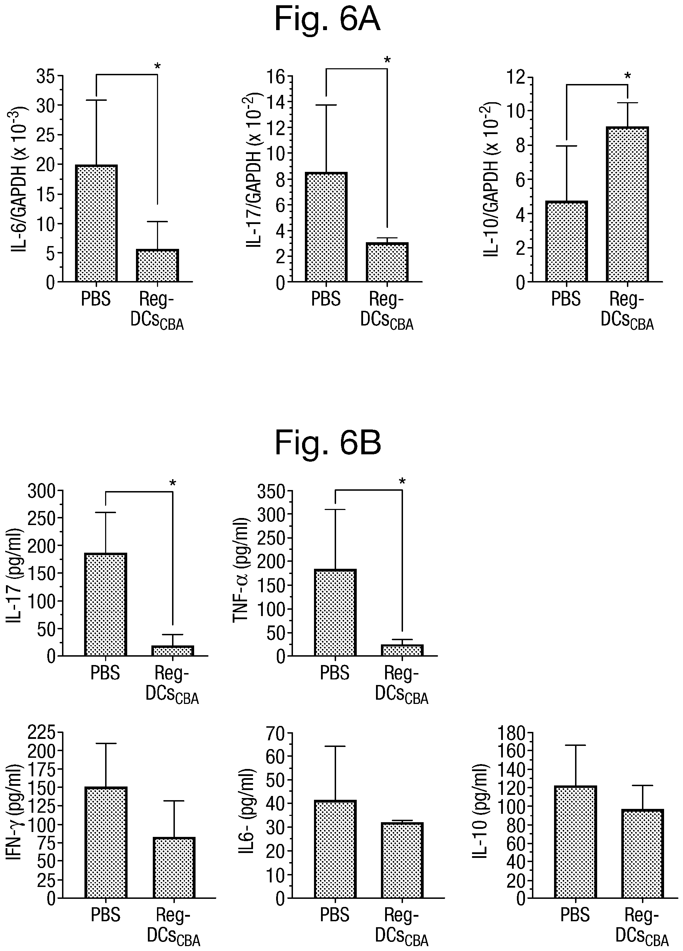

Fig. 6 is a graph of expression of inflammatory cytokine in colon of a colitis model mouse induced by CD4⁺CD25⁻ T cell. The results are shown in an average value ± SD. In the horizontal axis of Fig. 6, PBS indicates a mouse with transplanted CD4⁺CD25⁻ T cell which was administered with PBS; and Reg-DC_{CBA} indicates a mouse with transplanted CD4⁺CD25⁻ T cell which was administered with CBA-pulsed regulatory dendritic cell. A single asterisk means P < 0.05 compared with the mouse which was administered with PBS. Fig. 6A is a graph showing expression of cytokine mRNA in the colon quantitated by real time RT-PCR at 4 weeks after the cell transplantation of CD4⁺CD25⁻ T cell. In Fig. 6A, the vertical axis indicates proportion of an expression level of each cytokine mRNA relative to a level of mRNA of GAPDH. Fig. 6B is a graph showing concentration of cytokine measured by ELISA and a Cytometric Bead Array (BD Biosciences Pharmingen)in culture supernatant from colon cells cultured for 3 days, wherein the seeded colon cells were taken at 4 weeks after cell transplantation of CD4⁺CD25⁻ T cell. In Fig. 6B, the vertical axis indicates concentration of each cytokine in pg/mL.

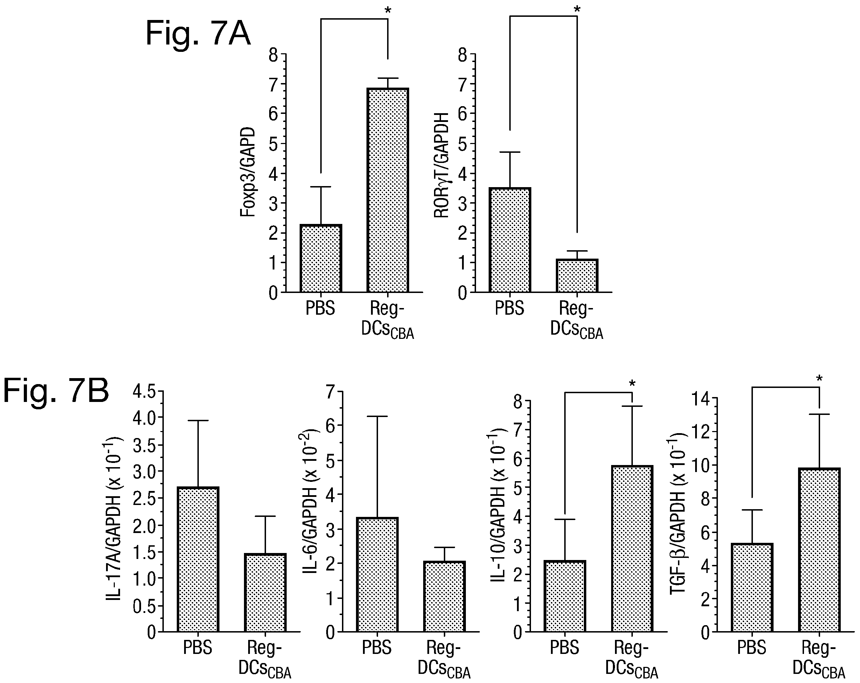

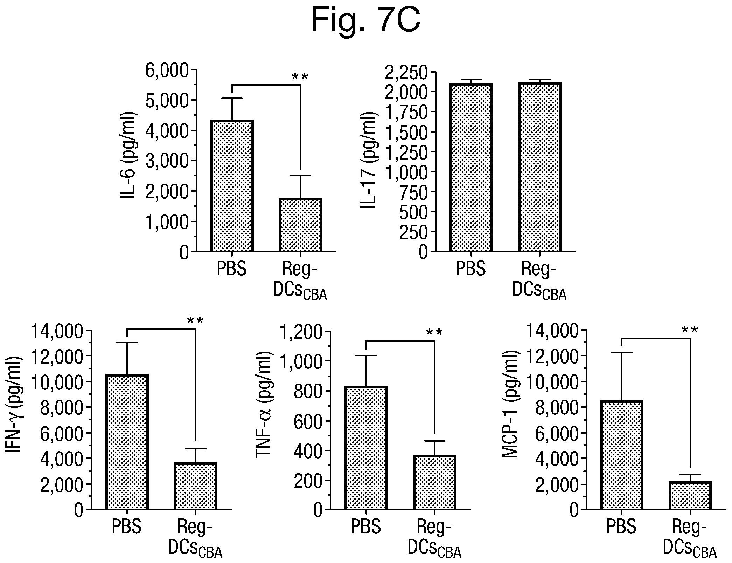

Fig. 7 is a graph showing expression of transcription factors and inflammatory cytokines in mesenteric lymphocytes (hereinafter referred to as "MLN,") of a colitis model mouse induced by the CD4⁺CD25⁻ T cell. The results are shown in an average value ± SD. In the horizontal axis of Fig. 7, PBS means a mouse with transplanted CD4⁺CD25⁻ T cell which was administered with PBS; and Reg-DC_{CBA} means a mouse with transplanted CD4⁺CD25⁻ T cell which was administered with CBA-pulsed regulatory dendritic cell. A single asterisk means P < 0.05 compared with the mouse which was administered with PBS, and a double asterisk means P < 0.01 compared with the mouse which was administered with PBS. Fig. 7A is a graph showing expression of a transcription factor mRNA in the MLN quantitated by real time RT-PCR at 4 weeks after the cell transplantation of the CD4⁺CD25⁻ T cell. In Fig. 7A, the vertical axis indicates proportion of an expression level of each transcription factor mRNA relative to a level of mRNA of GAPDH. Fig. 7B is a graph showing expression of cytokine mRNA in the MLN quantitated by real time RT-PCR at 4 weeks after the cell transplantation of the CD4⁺CD25⁻ T cell. In Fig. 7B, the vertical axis indicates proportion of an expression level of each cytokine mRNA relative to a level of mRNA of GAPDH. Fig. 7C is a graph of concentration of cytokine measured by ELISA and a Cytometric Bead Array (BD Biosciences Pharmingen) in culture supernatant obtained by incubating MLN in the presence of 25 ng/mL of PMA and 1 µg/mL of ionomycin for 72 hours, wherein the seeded MLN was taken at 4 weeks after the cell transplantation of the CD4⁺CD25⁻ T cell. In Fig. 7C, the vertical axis indicates concentration of each cytokine in pg/mL.

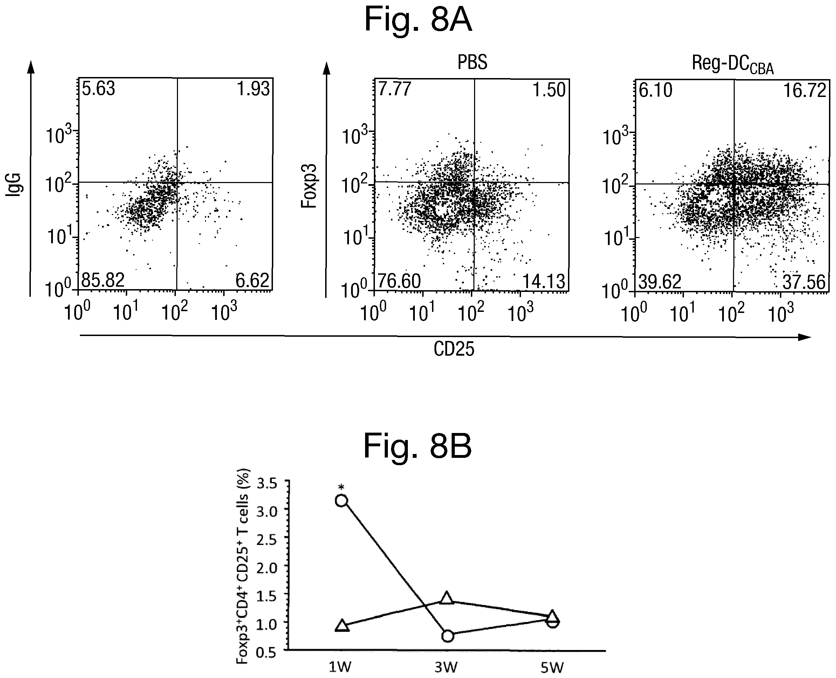

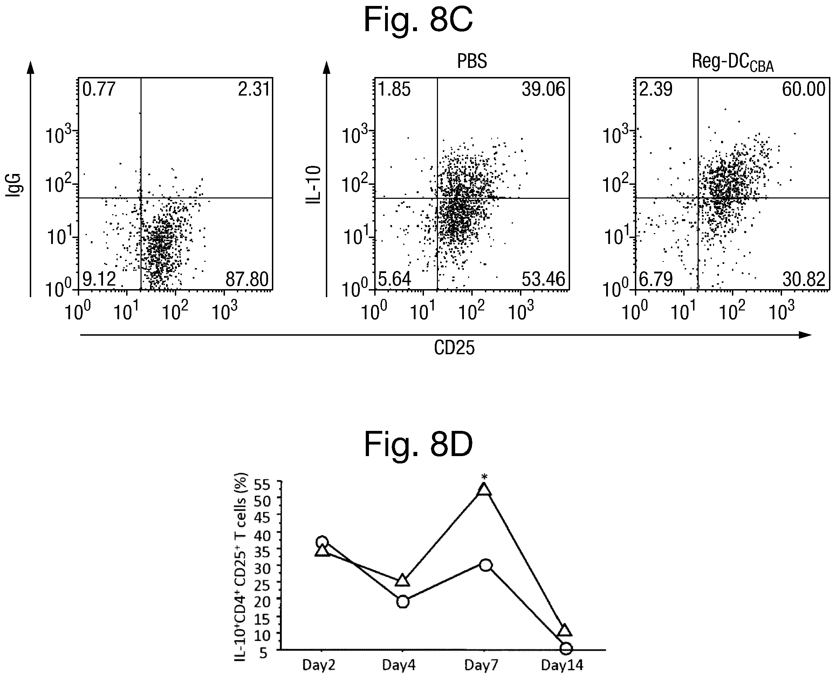

Fig. 8 is a graph showing in vivo induction of Foxp3⁺CD4⁺CD25⁺ T cell by administration of PBS or CBA-pulsed regulatory dendritic cell to the model mouse reconstituted with CD4⁺CD25⁻ T cell. Fig. 8A is a dot-plot showing results of flow cytometry analysis of the MLN taken at one week after the cell transplantation of the CD4⁺CD25⁻ T cell. The leftmost plot shows expression of an isotypic control IgG and CD25 in a BALB/C splenic cell analyzed by flow cytometry. In the leftmost plot, the vertical axis indicates expression level of isotypic control IgG, and the horizontal axis indicates expression level of CD25. "PBS" shows expression of Foxp3 and CD25 analyzed by flow cytometry after administration of PBS, wherein vertical axis indicates expression level of Foxp3, and the horizontal axis indicates expression level of CD25. "Reg-DC_{CBA}" shows expression of Foxp3 and CD25 analyzed by flow cytometry after administration of CBA-pulsed regulatory dendritic cell, wherein vertical axis indicates expression level of Foxp3, and the horizontal axis indicates expression level of CD25. Fig. 8B is a graph showing percentage of Foxp3⁺CD4⁺CD25⁺ T cells in the MLN taken at 1, 3 and 5 weeks after cell transplantation of CD4⁺CD25⁻ T cell. A single asterisk means P < 0.01 compared with the mouse which was administered with PBS. In Fig. 8B, an open circle indicates a mouse which was administered with CBA-pulsed regulatory dendritic cell; and an open triangle indicates a mouse which was administered with PBS. The vertical axis indicates percentage of Foxp3⁺CD4⁺CD25⁺ T cells in %, and the horizontal axis indicates a period of time in week(s) after cell transplantation of CD4⁺CD25⁻ T cell. Fig. 8C is a dot-plot showing results of flow cytometry of e MLN taken at seven days after cell transplantation of CD4⁺CD25⁻ T cell. The leftmost plot shows expression of the isotypic control IgG and CD25 in a BALB/C splenic cell analyzed by flow cytometry. In the leftmost plot, the vertical axis indicates expression level of the isotypic control IgG, and the horizontal axis indicates expression level of CD25. "PBS" shows expression of IL-10 and CD25 analyzed by flow cytometry after administration of PBS, wherein the vertical axis indicates expression level of IL-10, and the horizontal axis indicates expression level of CD25. "Reg-DC_{CBA}" shows expression of IL-10 and CD25 analyzed by flow cytometry after administration of CBA-pulsed regulatory dendritic cell, wherein the vertical axis indicates expression level of IL-10, and the horizontal axis indicates expression level of CD25. Fig. 8D is a graph showing percentage of IL-10⁺CD4⁺CD25⁺ T cells in MLNs taken at 2, 4, 7 and 14 days after cell transplantation of CD4⁺CD25⁻ T cell. A single asterisk means P < 0.01 compared with the mouse which was administered with PBS. In Fig. 8D, an open circle indicates a mouse which was administered with CBA-pulsed regulatory dendritic cell; and an open triangle indicates a mouse which was administered with PBS. The vertical axis indicates the percentage (%) of IL-10⁺CD4⁺CD25⁺ T cell, and the horizontal axis indicates a period of time (day(s)) after cell transplantation of CD4⁺CD25⁻ T cell.

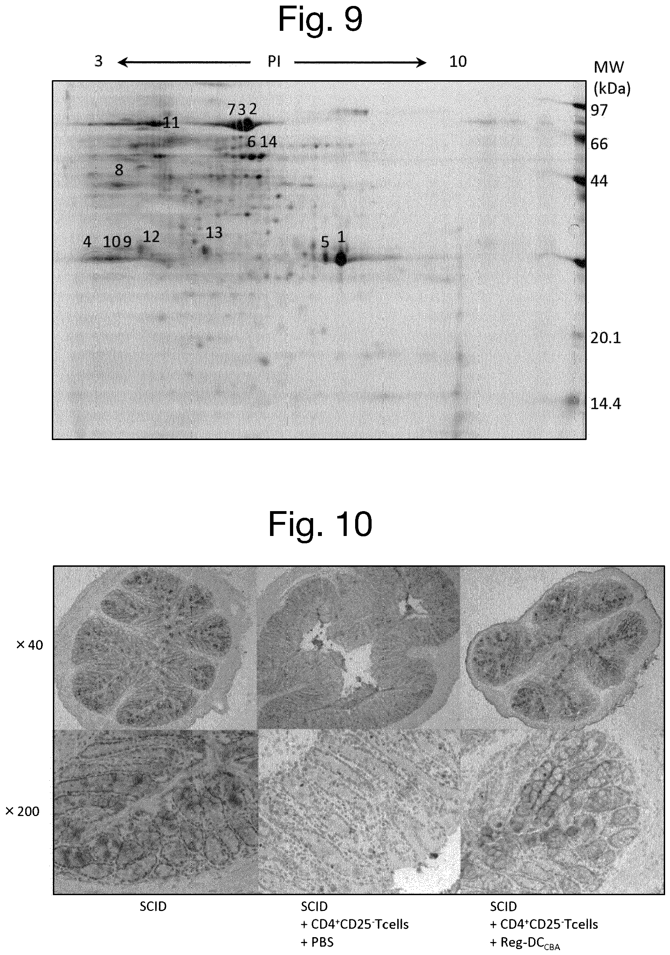

Fig. 9 is a photograph of two-dimensional electrophoresis gel of CBA stained with Deep Purple. Spots were labeled according to the spot number in Table 1. The number on the right side indicates molecular weight of the marker (kDa). The number on the top indicates a pH level of isoelectric focusing electrophoresis.
Fig. 10 is a photograph of immunohistochemical staining detecting expression of CA I in mouse colon tissue. In Fig. 10, the upper photograph shows an enlarged image at a magnification of 40 times, and the lower photograph shows an enlarged image at a magnification of 200 times. In order from left to right, "SCID" indicates a mouse without transplanted CD4⁺CD25⁻ T cell; "SCID + CD4⁺CD25⁻ T cells + PBS" indicates a mouse transplanted CD4⁺CD25⁻ T cell which was administered with PBS; and "SCID + CD4⁺CD25⁻ T cells + Reg-DC_{CBA}" indicates a mouse transplanted CD4⁺CD25⁻ T cell which was administered with CBA-pulsed regulatory dendritic cell.

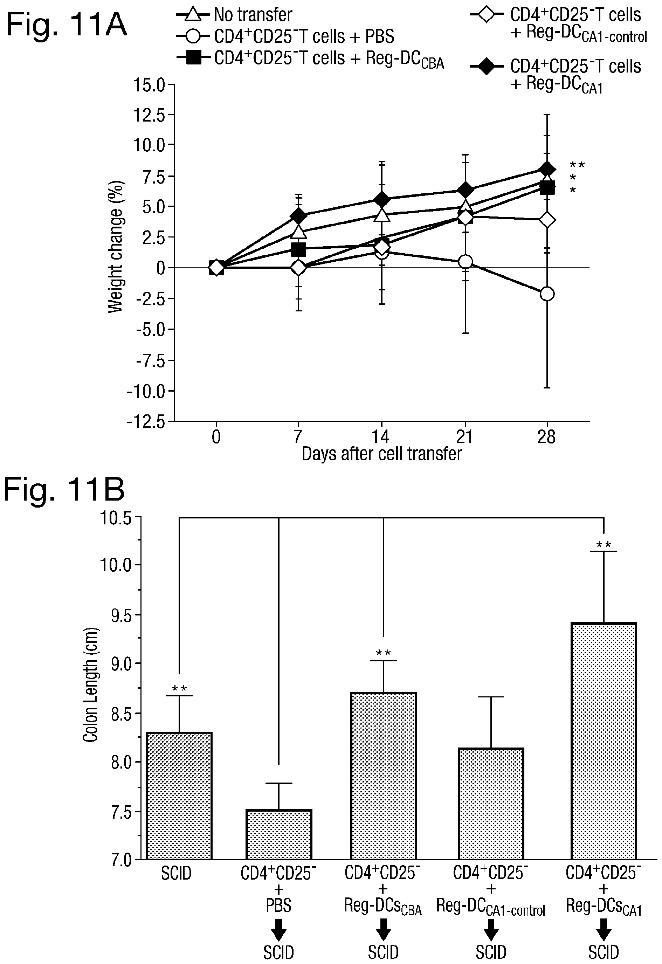

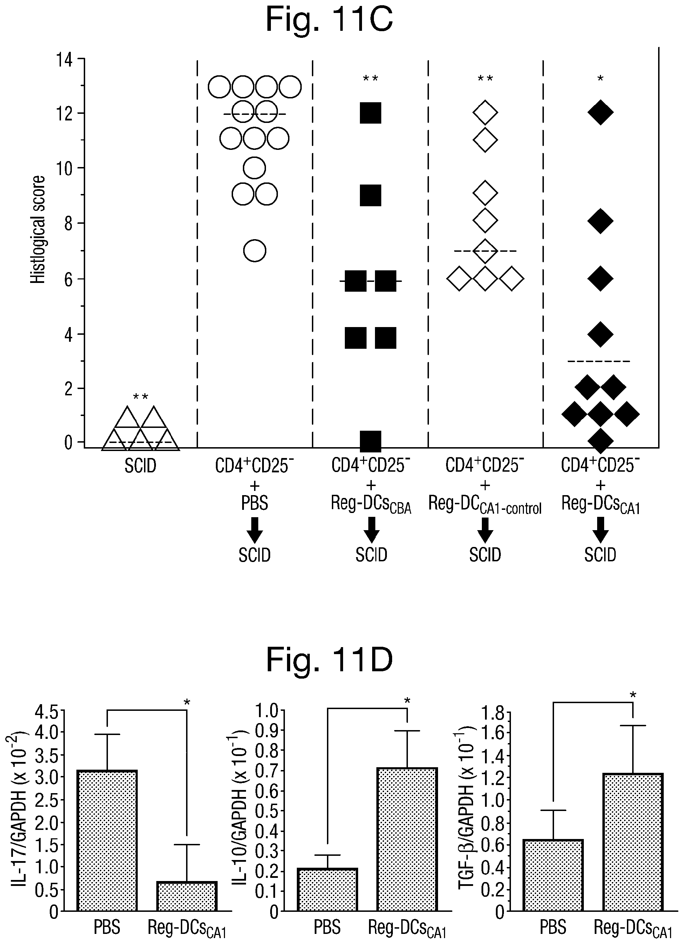

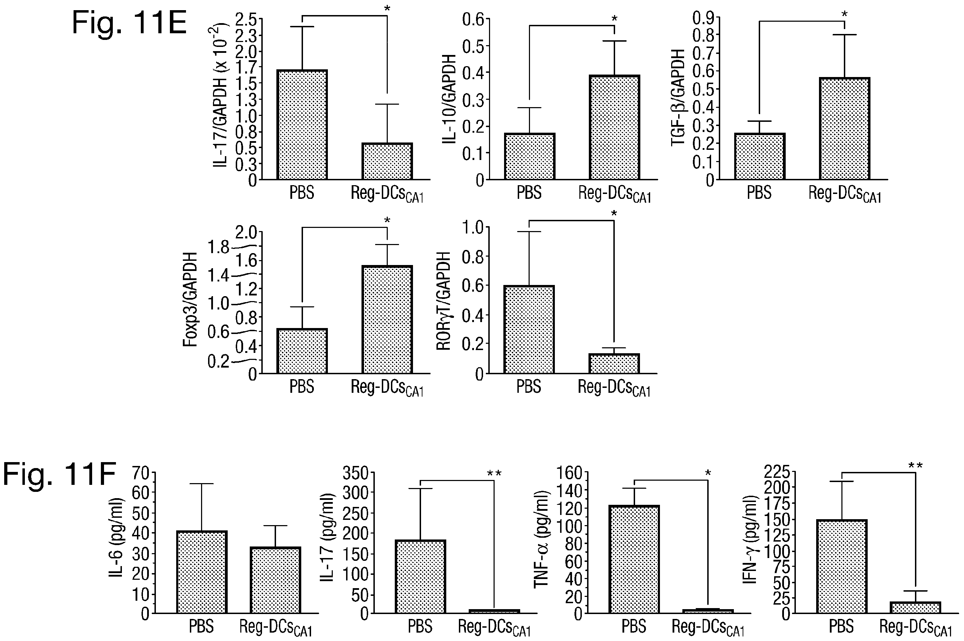

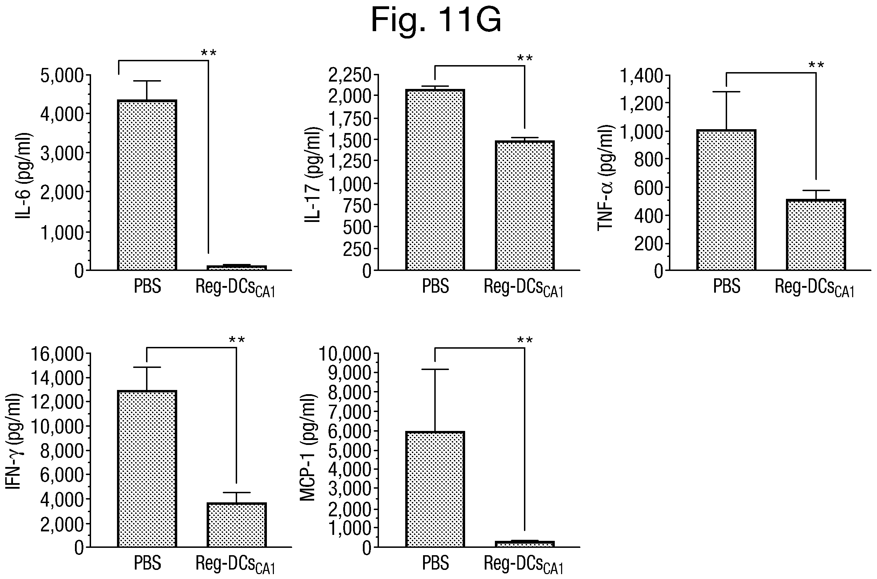

Fig. 11 shows results administration of CA I-pulsed regulatory dendritic cell to colitis model mouse induced by CD4⁺CD25⁻ T cell. On the horizontal axis of Figs. 11B and 11C, "SCID" indicates a mouse transplanted CD4⁺CD25⁻ T cell; "SCID + CD4⁺CD25⁻ T cells + PBS" indicates a mouse transplanted CD4⁺CD25⁻ T cell which was administered with PBS; "SCID + CD4⁺CD25⁻ T cells + Reg-DC_{CBA}" indicates a mouse with transplanted CD4⁺CD25⁻ T cell which was administered with CBA-pulsed regulatory dendritic cells; "SCID + CD4⁺CD25⁻ T cells + Reg-DC_{CA 1-control}" indicates a mouse which was administered with CA I-control-pulsed regulatory dendritic cells, wherein the CA I-control was synthesized using empty plasmid non-genetically recombined with gene sequence of CA I in the same method of synthesis of CA I which employs the cell free protein synthesis system; and "SCID + CD4⁺CD25⁻ T cells + Reg-DC_{CA 1}" indicates a mouse with transplanted with CD4⁺CD25⁻ T cell which was administered with CA I-pulsed regulatory dendritic cell. On the horizontal axis of Figs. 11D to 11G, "PBS" indicates a mouse with transplanted CD4⁺CD25⁻ T cell which was administered with PBS; "Reg-DC_{CA 1}" indicates a mouse with transplanted CD4⁺CD25⁻ T cell which was administered with CA I-pulsed regulatory dendritic cell. Fig. 11A is a graph of time course of change in body weight change (%). In Fig. 11A, the vertical axis indicates body weight change (%), and the horizontal axis indicates a number of day(s) after the cell transplantation. In Fig. 11A, a triangle indicates a mouse without transplanted CD4⁺CD25⁻ T cell; an open circle indicates a mouse with transplanted CD4⁺CD25⁻ T cell which was administered with PBS; a filled square indicates a mouse transplanted CD4⁺CD25⁻ T cell which was administered with CBA-pulsed regulatory dendritic cells; an open diamond indicates a mouse which was administered with CA I-control-pulsed regulatory dendritic cell, wherein the CA I-control was synthesized using empty plasmid non-genetically recombined with gene sequence of CA I in the same method of synthesis of CA I which employs the cell free protein synthesis system; a filled diamond indicates a mouse with transplanted CD4⁺CD25⁻ T cell which was administered with CA I-pulsed regulatory dendritic cell. An error bar indicates SD. A single asterisk means P < 0.05 compared with mouse which was administered with PBS, and a double asterisk means P < 0.01 compared with the mouse which was administered with PBS. Fig. 11B is a graph of length of colon at 28 days after the cell transplantation of the CD4⁺CD25⁻ T cell. In Fig. 11B, the vertical axis indicates length (cm) of colon, and the horizontal axis indicates mouse used for length measurement of colon. An error bar indicates SD. A double asterisk means P < 0.01 compared with the mouse which was administered with PBS. Fig. 11C is a graph of calculated histological scores. In Fig. 11C, the vertical axis indicates a histological score, and the horizontal axis indicates mouse used for the histological scoring. A horizontal line therein indicates a median value. A single asterisk means P < 0.05 compared with the mouse which was administered with PBS, and a double asterisk means P < 0.01 compared with the mouse which was administered with PBS. Fig. 11D is a graph of expression of inflammatory cytokine mRNA in colon quantitated by real time RT-PCR at 4 weeks after cell transplantation of CD4⁺CD25⁻ T cell. The results are shown in an average value ± SD. In Fig. 11D, the vertical axis indicates proportion of an expression level of each cytokine mRNA relative to a level of mRNA of GAPDH. A single asterisk means P < 0.05 compared with the mouse which was administered with PBS. Fig. 11E is a graph of expression of a transcription factor and inflammatory cytokine mRNA in the MLN quantitated by real time RT-PCR at 4 weeks after cell transplantation of CD4⁺CD25⁻ T cell. The results are shown in an average value ± SD. In Fig. 11E, the vertical axis indicates proportion of an expression level of transcription factor mRNA and each cytokine mRNA relative to a level of mRNA of GAPDH. A single asterisk means P < 0.05 compared with the mouse which was administered with PBS. Fig. 11F is a graph showing concentration of cytokine measured by ELISA in culture supernatant from colon cells cultured ex vivo for 3 days, wherein the seeded colon cells was taken at 4 weeks after cell transplantation of CD4⁺CD25⁻ T cell. The results are shown in an average value ± SD. In Fig. 11F, the vertical axis indicates concentration (pg/mL) of each cytokine. A single asterisk means P < 0.05 n compared with the mouse which was administered with PBS. A double asterisk means P < 0.01 compared with the mouse which was administered with PBS. Fig. 11G is a graph showing concentration of cytokine measured by ELISA and a Cytometric Bead Array (BD Biosciences Pharmingen) in a culture supernatant obtained by incubating MLN in the presence of 25 ng/mL of PMA and 1 µg/mL of ionomycin for 72 hours, wherein the seeded MLN was taken at 4 weeks after cell transplantation of CD4⁺CD25⁻ T cell. The results are shown in an average value ± SD. In Fig. 11G, the vertical axis indicates concentration (pg/mL) of each cytokine. A single asterisk herein means P < 0.05 compared with the mouse which was administered with PBS. A double asterisk means P < 0.01 compared with the mouse which was administered with PBS.

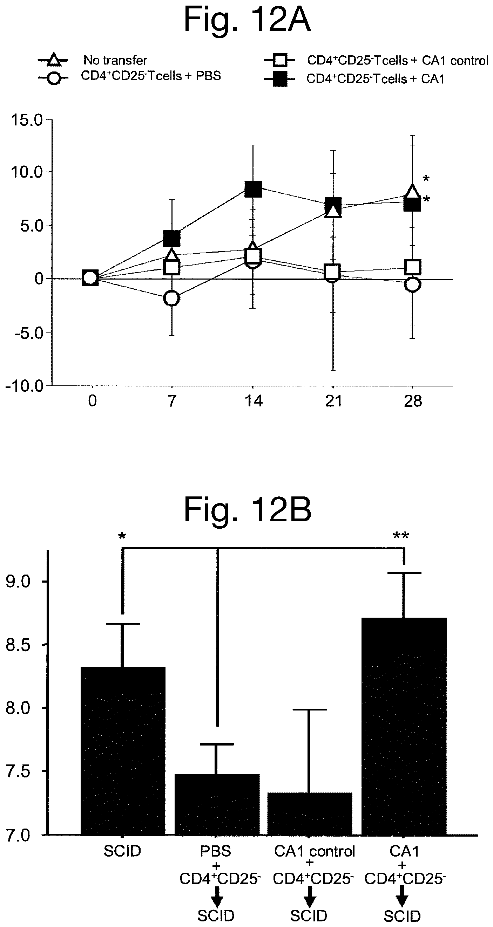

Fig. 12A is a graph showing data of model mice induced enteritis by transplanting CD4⁺CD25⁻ T cells after administration of PBS, mCA 1-control or mCA 1 (also in Figs. 12B to 12G). Fig. 12A shows time course of change in body weight (%). In Fig. 12A, the vertical axis indicates the body weight change (%), and the horizontal axis indicates a number of day(s) after the cell transplantation. In Fig. 12A, a triangle indicates SCID mice (n=6) without transplanted CD4⁺CD25⁻ T cell; an open circle indicates SCID mice (n=7) which were transplanted CD4⁺CD25⁻ T cells after administration of PBS; an open square indicates SCID mice (n=7) which were transplanted CD4⁺CD25⁻ T cells after administration of a protein (mCA 1-control) synthesized from empty plasmid non-genetically recombined with gene sequence of CA I; and a filled square indicates SCID mice (n=7) which were transplanted CD4⁺CD25⁻ T cells after administration of mCA 1. An error bar indicates SD. A single asterisk means P < 0.05 compared with the mice which were administered with PBS.

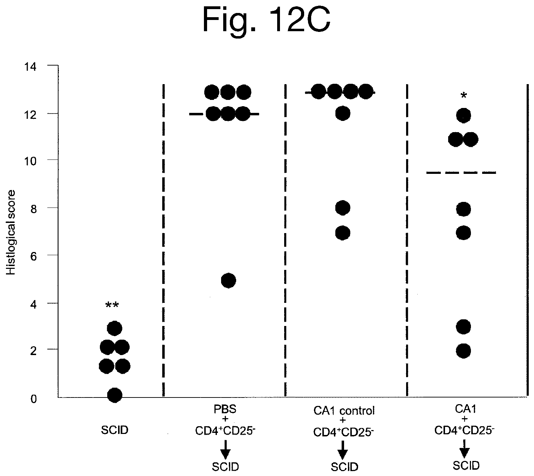

Fig. 12B is a graph of colon length at 28 days after the cell transplantation of the CD4⁺CD25⁻ T cells. In Fig. 12B, the vertical axis indicates a length (cm) of colon, and the horizontal axis indicates type of mouse measured colon length. On the horizontal axis, "SCID" indicates a mouse without transplanted CD4⁺CD25⁻ T cell; "PBS + CD4⁺CD25⁻ → SCID" indicates a mouse which was transplanted CD4⁺CD25⁻ T cells after administration of PBS; "CA 1-control + CD4⁺CD25⁻ → SCID" indicates a mouse which was transplanted CD4⁺CD25⁻ T cells after administration of a protein (mCA 1-control) synthesized from empty plasmid non-genetically recombined with gene sequence of CA I; and "CA 1 + CD4⁺CD25⁻" indicates a mouse which was transplanted CD4⁺CD25⁻ T cells after administration of mCA 1 (also in Fig. 12C.) An error bar indicates SD. Each group consists of 6 to 7 mice. A single asterisk means P < 0.05 compared with the mice which were administered with PBS, and a double asterisk means P < 0.01 compared with the mice which were administered with PBS.
Fig. 12C is a graph of histological scores of colon from CD4⁺CD25⁻ T cell-transplanted enteritis model mice which were administered with PBS, mCA1-control or mCA1. In Fig. 12C, the vertical axis indicates a histological score, and the horizontal axis indicates type of mouse used for the histological scoring. A horizontal line therein indicates a median value. Each group consists 6 to 7 mice. A single asterisk means P < 0.05 compared with the mice which were administered with PBS.

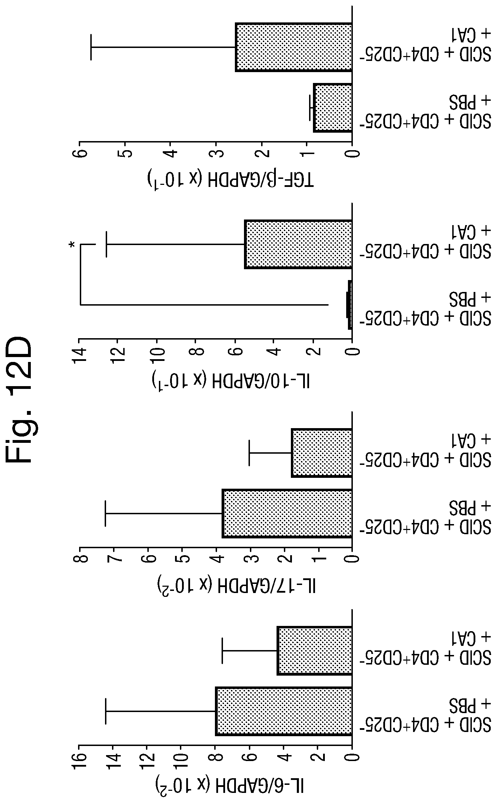

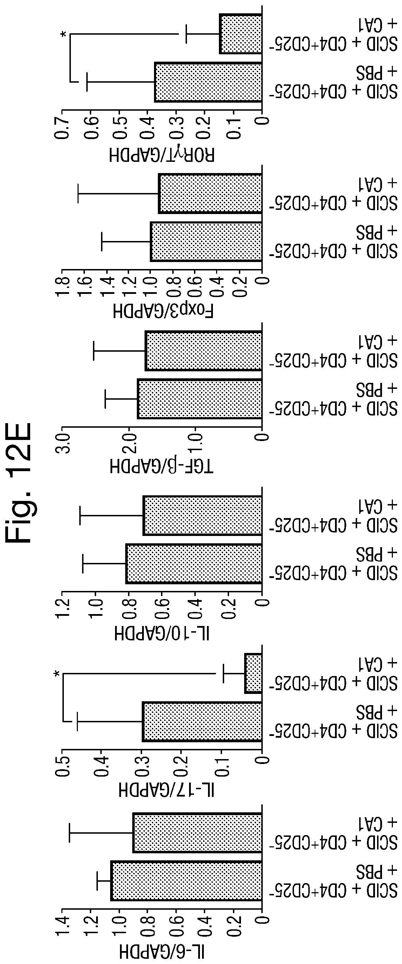

Fig. 12D is a graph of expression of inflammatory cytokine mRNA in colon quantitated by real time RT-PCR at 4 weeks after cell transplantation of CD4⁺CD25⁻ T cells. The results are shown in an average value of five mice ± SD. In Fig. 12D, the vertical axis indicates proportion of an expression level of each cytokine mRNA relative to a level of mRNA of GAPDH. On the horizontal axis, "SCID + CD4⁺CD25⁻ + PBS" indicates a mouse which was transplanted CD4⁺CD25⁻ T cells after administration of PBS; and "SCID + CD4⁺CD25⁻ + CA 1" indicates a mouse which was transplanted CD4⁺CD25⁻ T cells after administration of CA 1 (also in Figs. 12E to 12G). A single asterisk means P < 0.05 compared with the mice which were administered with PBS.
Fig. 12E is a graph of expression of a transcription factor mRNA and inflammatory cytokine mRNA in MLN quantitated by real time RT-PCR at 4 weeks after cell transplantation of CD4⁺CD25⁻ T cell. The results are shown in an average value of five mice ± SD. In Fig. 12E, the vertical axis indicates proportion of an expression level of each transcription factor mRNA and each cytokine mRNA relative to a level of mRNA of GAPDH. A single asterisk means P < 0.05 compared with the mice which were administered with PBS.

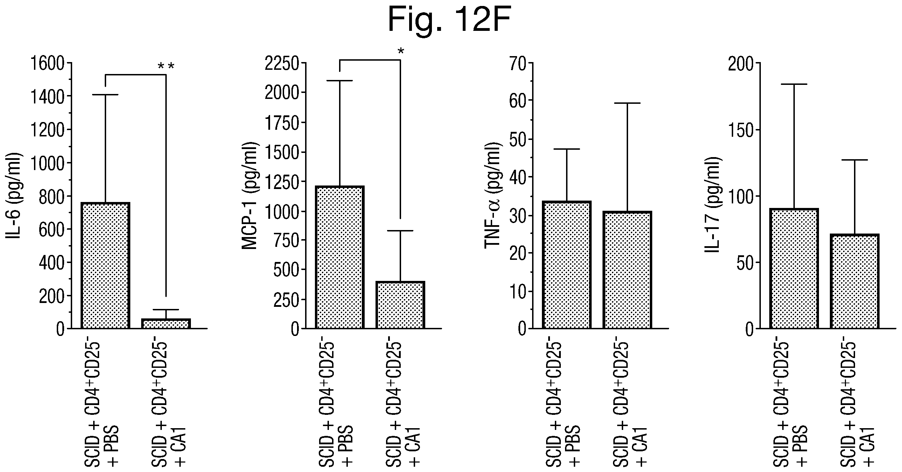

Fig. 12F is a graph of concentration of IL-6, MCP-1, TNFα and IL-17 measured by a Cytometric Bead Array (BD Biosciences Pharmingen) and ELISA (R&D Systems) which were secreted in culture supernatant from colon cells cultured ex vivo for 3 days. The results are shown in an average value ± SD. In Fig. 12F, the vertical axis indicates concentration (pg/mL) of each cytokine. Each group consists of 5 to 7 mice. A single asterisk means P < 0.05 compared with the mice which were administered with PBS. A double asterisk means P < 0.01 compared the mice which were administered with PBS.

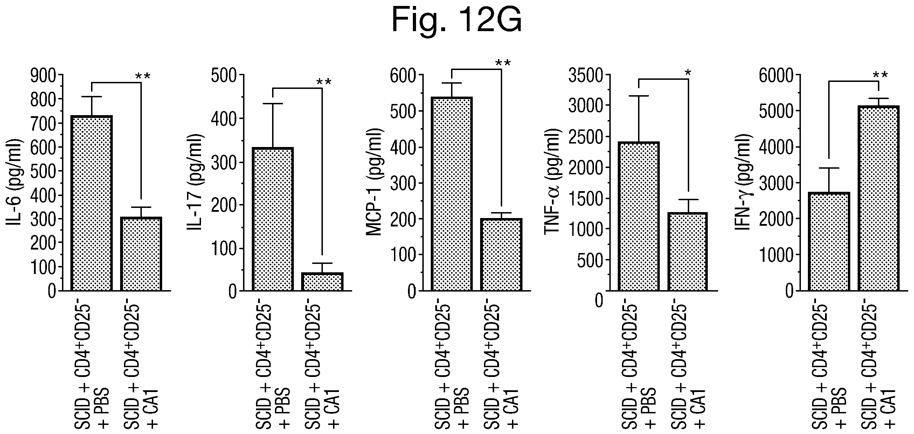

Fig. 12G is a graph of concentration of IL-6, MCP-1, TNFα, IFNγ and IL-17 measured by a Cytometric Bead Array (BD Biosciences Pharmingen) and ELISA (R&D Systems) which were secreted in culture supernatant obtained by incubating MLN (1 x 10⁶ cells) in the presence of 25 ng/mL of PMA and 1 µg/mL of ionomycin for 72 hours. The results are shown in an average value ± SD. In Fig. 12G, the vertical axis indicates concentration (pg/mL) of each cytokine. Each group consists of 5 to 7 mice. A single asterisk means P < 0.05 compared with the mice which were administered with PBS; and a double asterisk means P < 0.01 compared with the mice which were administered with PBS.

### [Description of Embodiments]

CA I may be prepared by appropriately designing a primer with reference to the gene sequence information well known to those skilled in the art (for example, the gene sequence of mouse CA I is shown as SEQ ID No. 1, and the gene sequence of human CA I is shown as SEQ ID No. 3), generating an expression vector and transforming the vector into Bacillus coli, fermentum, insect cells, animal cells and the like to induce the expression of the CA I. Alternatively, CA I may be also prepared employing cell free protein synthesis system using wheat germ ribosomal RNA (K. Madin et al., Proc. Natl. Acad. Sci. USA, 97: 559-64 (2000)).

In using a partial peptide of CA I as the CA I, it may be prepared by method for amino acid synthesis which is well known to those skilled in the art. For instance, the partial peptide of the CA I may be prepared by using chemical synthesis method, such as Fmoc method or Boc method, or by using an automated peptide synthesizer. Such peptide synthesizer includes, for example, PSSM-8 (Shimadzu Corporation); model 433A Peptide Synthesizer (Applied Biosystems, Inc.); ACT396Apex (Advanced ChemTech Inc.) and the like.

Introduction of mutation into the CA I (including its partial peptide) may be performed using the technique well known to those skilled in the art, such as site-specific mutagenesis.

"The CA I for use in a method for inducing immune tolerance in a patient" according to the present invention encompasses direct administration of CA I into the patient, and an *ex vivo* pulse of a tolerogenic antigen presentation cell by CA I.

"The CA I for use in a method for treating or preventing an inflammatory bowel disease" according to the present invention encompasses direct administration of CA I into the patient, and an *ex vivo* pulse of a tolerogenic antigen presentation cell by CA I.

In the present invention, CA I or a pharmaceutical composition comprising CA I as an active ingredient into a patient may be directly administered via routes, such as oral, nasal, intratracheal, subcutaneous, intravascular (i.e., intravenous) routes and the like. Most preferable administration route is oral administration. A drug formulation for the composition may include, for example, an injection, capsule, tablet, syrup, granule, powder-fog agent and the like. The administration is not particularly limited as long as the desirable therapeutic or preventive effect can be obtained. Most preferable administration is oral administration. Further, the administration may be performed temporarily, or continuously or intermittently. For instance, the administration frequency may be selected from, for example, single administration, once to 4 times a week, and intermittent dosing such as once a month to every three months. A dosage is not particularly limited as long as the desirable therapeutic or preventive effect can be obtained, and may be appropriately determined depending on a symptom, sex, age and the like. Further, the dosage may be determined based on, for example, a yield of the antigen-specific tolerogenic antigen presentation cells or antigen-specific regulatory T cells, or degree of therapeutic or preventive effect on the inflammatory bowel disease. For instance, a dosage of the antigen may be 0.01 ng/kg to 10 mg/kg, preferably 0.1 ng/kg to 1 mg/kg, more preferably 0.5 ng/kg to 100 µg/kg, and the most preferably 1 ng/kg to 10 µg/kg. Furthermore, CA I may be administered together with other pharmaceutical agents including an immunosuppressive agent such as cyclosporine and tacrolimus and the like. For example, CA I and such other pharmaceuticals may be administered at once, or separately at an interval.

The above described method for inducing immune tolerance, comprising *ex vivo* pulse of tolerogenic antigen presentation cell by CA I may be performed, for example, by the following steps:
a step of preparing tolerogenic antigen presentation cell;
a step of pulsing the tolerogenic antigen presentation cell by CA I; and
a step of administering the pulsed tolerogenic antigen presentation cell into a patient who is subjected to be induced immunological tolerance.

Preferably, the method for inducing immune tolerance, comprising *ex vivo* pulse of the tolerogenic antigen presentation cell by CA I according to the present invention may also be performed, for example, by the following steps:
a step of preparing a patient-derived regulatory dendritic cell;
a step of pulsing the regulatory dendritic cell by CA I; and
a step of administering the pulsed regulatory dendritic cell into the patient who is subjected to be induced immunological tolerance induction.

The above described method for treating or preventing inflammatory bowel disease, comprising *ex vivo* pulse of the tolerogenic antigen presentation cell by CA I may be performed, for example, by the following steps:
a step of preparing the tolerogenic antigen presentation cell;
a step of pulsing the tolerogenic antigen presentation cell by CA I; and
a step of administering the pulsed tolerogenic antigen presentation cell into the patient of the inflammatory bowel disease.

Preferably, the method for treating or preventing an inflammatory bowel disease, comprising ex vivo pulse of tolerogenic antigen presentation cell by CA I may also be performed by the following steps:
a step of preparing a patient-derived regulatory dendritic cell;
a step of pulsing the regulatory dendritic cell by CA I; and
a step of administering the pulsed regulatory dendritic cell into the patient of the inflammatory bowel disease.

The regulatory dendritic cell, which is one of the tolerogenic antigen presentation cell, may be prepared by taking bone marrow cells form the patient, incubating the cells in presence of GM-CSF, IL-10 and a human transforming growth factor-β1 (TGF-β1) for 8 days and then stimulating the cells in the presence of an ultrapure LPS for 24 hours. Alternatively, the regulatory dendritic cell may be prepared by taking a peripheral blood, isolating and collecting a fraction of mononuclear cells using methods well known to those skilled in the art, incubating the cells in the presence of GM-CSF, IL-10 and a human transforming growth factor-β1 (TGF-β1) for 8 days and then stimulating the cells in the presence of the ultrapure LPS for 24 hours. The resulting regulatory dendritic cell may be further purified by removing dendritic cells expressing costimulatory molecules.

Pulse of the tolerogenic antigen presentation cell by CA I may be performed by incubating the tolerogenic antigen presentation cells in the presence of CA I.

Administration of the pulsed tolerogenic antigen presentation cell into the patient may be performed using well known technique to those skilled in the art as cell therapy employing immunocyte. For example, the cell may be administered into the blood vessel (the intravenous). The administration may be performed at once, or continuously or intermittently. For instance, the administration may be performed preferably as single administration or once to 4 times a week, and more preferably as single administration or once a week. The intermittent administration, such as once a month to every three months, may be also employed. A dose is not particularly limited as long as the desirable therapeutic or preventive effect can be obtained, and may be appropriately determined depending on a symptom, sex, age and the like. The dose may be determined based on, for example, degree of the immunological tolerance, number of the antigen-specific regulatory dendritic cell and/or the regulatory T cell, or degree of the therapeutic or preventive effect on the inflammatory bowel disease. For instance, a dose of the cells may be 1 x 10⁶ cells to 1 x 10¹⁰ cells, preferably 1 x 10⁷ cells to 1 x 10⁹ cells, more preferably 5 x 10⁷ cells to 5 x 10⁸ cells, most preferably 1 x 10⁸ cells. The pulsed tolerogenic antigen presentation cell may be administered together with other pharmaceuticals, including an immunosuppressive agent such as cyclosporine and tacrolimus and the like. For example, the cells and such other pharmaceuticals may be administered at once, or separately at an interval.

The method for inducing immune tolerance, comprising *ex vivo* pulse of the tolerogenic antigen presentation cell by CA I, and the method for treating or preventing inflammatory bowel disease, comprising *ex vivo* pulse of the tolerogenic antigen presentation cell by CA I may further comprise a step of include *ex vivo* inducing the CA I-specific regulatory T cells *ex vivo.*

The method for inducing immune tolerance, comprising ex vivo pulse of the tolerogenic antigen presentation cell by CA I may be performed, for example, by the following steps:
a step of preparing tolerogenic antigen presentation cells and naive T cells;
a step of pulsing the tolerogenic antigen presentation cells by CA I;
a stop of contacting the pulsed tolerogenic antigen presentation cells with the naive T cells to induce the regulatory T cells; and
a step of administering the induced regulatory T cells into the patient who is subjected to induce immunological tolerance.

Preferably, the method for inducing immune tolerance, comprising ex vivo pulse of the tolerogenic antigen presentation cell by CA I may be performed, for example, by the following steps:
a step of preparing a patient-derived regulatory dendritic cells and naive T cells;
a step of pulsing the regulatory dendritic cells by CA I; and
a step of contacting the pulsed regulatory dendritic cells with the naive T cells to induce the regulatory T cells; and
a step of administering the induced regulatory T cells into the patient who is subjected to induce immunological tolerance.

The method for treating or preventing inflammatory bowel disease, comprising ex vivo pulse of the tolerogenic antigen presentation cells by CA I may be performed, for example, by the following steps:
a step of preparing tolerogenic antigen presentation cells and a naive T cells;
a step of pulsing the tolerogenic antigen presentation cells by CA I;
a step of contacting the pulsed tolerogenic antigen presentation cells with the naive T cells to induce the regulatory T cells; and
a step of administering the induced regulatory T cells into the patient of the inflammatory bowel disease.

Preferably, the method for treating or preventing an inflammatory bowel disease, comprising ex vivo pulse of the tolerogenic antigen presentation cells by CA I may be performed by the following steps:
a step of preparing patient-derived regulatory dendritic cells and naive T cells;
a step of pulsing the regulatory dendritic cells by CA I;
a step of contacting the pulsed regulatory dendritic cells with the naive T cells to induce the regulatory T cells; and
a step of administering the induced regulatory T cells into the patient of the inflammatory bowel disease.

Said naive T cell may be isolated from peripheral blood or spleen cells with a CD4⁺CD25⁺ regulatory T cell Isolation Kit and AutoMACS (Miltenyi Biotec GmbH) according to the manufacturer's protocols. Purity of the naive T cells may be identified by FACS analysis using peridinin chlorophyll protein (PerCP) labeled anti-CD4 monoclonal antibody and phycoerythrin (PE) labeled anti-CD25 monoclonal antibody.

The induction of regulatory T cells by pulsed regulatory dendritic cells may be achieved, for example, by incubating 5 x 10⁶ of naive T cells together with 5 x 10⁵ of regulatory dendritic cells in 1 mL of RPMI medium for 7 days.

The administration of the induced regulatory T cells into the patient may be performed similarly to the above-described administration of the pulsed tolerogenic antigen presentation cells into the patient.

"The pharmaceutical composition for use in treatment or prevention of inflammatory bowel disease, comprising CA I as an active ingredient" of the present invention may be a composition which is directly administered into a patient to induce immunological tolerance, or may be a composition used to prepare the antigen-specific tolerogenic antigen presentation cells ex vivo. When the composition is used to prepare the antigen-specific tolerogenic antigen presentation cells, the resulting tolerogenic antigen presentation cells or the regulatory T cells stimulated by such antigen presentation cells can be administered into the patient in accordance with the above described methods.

"The pharmaceutical composition for use in treatment or prevention of an inflammatory bowel disease, comprising CA I-specific tolerogenic antigen presentation cells as an active ingredient" may be a composition which is directly administered into the patient to induce immunological tolerance, or may be a composition which is used to prepare the antigen-specific regulatory T cells ex vivo. The CA I-specific tolerogenic antigen presentation cell may be provided by preparing tolerogenic antigen presentation cells and then pulsing the tolerogenic antigen presentation cells by CA I in accordance with the above described methods. The resulting tolerogenic antigen presentation cells or the regulatory T cells stimulated by such antigen presentation cell may be administered into the patient in accordance with the above described methods.

"The pharmaceutical composition for use in treatment or prevention of inflammatory bowel disease, comprising CA I-specific regulatory T cells as an active ingredient" may be a composition which is directly administered into the patient to induce immunological tolerance. The CA I-specific regulatory T cells may be provided by taking tolerogenic antigen presentation cells and the naive T cells, pulsing the tolerogenic antigen presentation cells by CA I and then contacting the pulsed tolerogenic antigen presentation cells with the naive T cells. The resulting regulatory T cell may be administered into the patient in accordance with the above described methods.

For the present invention, the composition comprising CA I may appropriately contain additives such as a stabilizing agent (see, for example, "Pharmaceutical Excipients Dictionary" Yakuji Nippo Limited., and "Handbook of Pharmaceutical Excipients" APhA Publications), in addition to CA I. It may also contain other pharmaceuticals, including an immunosuppressive agent such as cyclosporine, tacrolimus and the like. In the present invention, the composition comprising tolerogenic antigen presentation cells or regulatory T cells may appropriately further contain suitable additives for the cell therapy, in addition to the tolerogenic antigen presentation cells or the regulatory T cells.

### [Statistical Analysis]

For all of individual experiments, data was reported in an average value ± SD. The data was examined by Student's t-test or Mann-Whitney's U test. The statistical significance was defined as P < 0.05. Statistic calculation was performed using StatView version 5.0 Statistical Program.

### Example 1: Detection of dendritic cell marker

### (1) Mouse

In all of following experiments, a C.B-17 SCID mouse mated under the specific pathogen-free condition and a BALB/c (H-2d) female mouse were employed (CLEA Japan, Inc., Tokyo, Japan). All of the mice were 8 to 12-week-old and were maintained by feeding conventional laboratory animal feed at 22 °C and 55 % relative humidity in a cycle of 12 hours light and 12 hours darkness. All animals bred according to Good Laboratory Practice Guidelines. This study was performed with approval from Laboratory Animal Care Committee of Ehime University.

### (2) Preparation of dendritic cell

The mature dendritic cells were prepared by incubating 2 x 10⁶ of bone-marrow cells taken from the BALB/c mouse in the presence of 20 ng/mL of mouse granulocyte macrophage-colony stimulating factor (GM-CSF) (Wako Pure Chemical Industries, Ltd., Osaka, Japan) for 8 days and then stimulating them in the presence of 1 µg/mL of the ultrapure LPS (InvivoGen, San Diego, CA, US) for 24 hours.

The regulatory dendritic cells were prepared by incubating 2 x 10⁶ of bone-marrow cells taken from the BALB/c mouse in the presence of 20 ng/mL of mouse GM-CSF (Wako Pure Chemical Industries, Ltd.), mouse interleukin-10 (IL-10) (Wako Pure Chemical Industries, Ltd.) and human transforming growth factor-β1 (TGF-β1) (Wako Pure Chemical Industries, Ltd.) for 8 days and then stimulating them in the presence of 1 µg/mL of the ultrapure LPS (InvivoGen) for 24 hours. Next, the regulatory dendritic cells were stained with a fluorescein isothiocyanate (FITC) labeled anti-CD40 monoclonal antibody (Clone 3/23, BD Biosciences Pharmingen, San Diego, CA, US), an anti-CD80 monoclonal antibody (Clone 16-10AI, BD Biosciences Pharmingen) and an anti-CD86 monoclonal antibody (Clone GL1, BD Biosciences Pharmingen) at 4 °C for 30 minutes. The stained cells were labeled with anti-FITC microbeads (Miltenyi Biotec GmbH, Bergisch Gladbach, Germany), and then, the CD40⁺CD80⁺CD86⁺ cell (approximately 20 % of the total cells prepared) was eliminated with AutoMACS (Miltenyi Biotec GmbH) to remove the dendritic cell expressing a costimulatory molecule.

### (3) Detection of dendritic cell maker

The dendritic cells were washed and then resuspended into a solution of 1 % FBS and 0.2 % sodium azide in PBS. After blocking its Fc receptor by a purified rat anti-mouse CD16/CD32 monoclonal antibody (Clone 2.4G2, BD Pharmingen), the dendritic cells were stained with a FITC labeled anti-CD11c monoclonal antibody (Clone HL3, BD Pharmingen), an anti-CD80 monoclonal antibody, an anti-CD86 monoclonal antibody and an anti-CD40 monoclonal antibody, a PE labeled anti-I-A/I-E monoclonal antibody (Clone 2G9, BD Pharmingen) and an anti-H-2K^{d} monoclonal antibody (Clone AMS-32.1, BD Pharmingen) in a dark room at 4 °C for 30 minutes. An isotype-matched monoclonal antibody was used as a control. The fluorescence staining was examined by the flow cytometry analysis.

### (4) Results

The results are shown in Fig. 1. The bone marrow-derived mature dendritic cells expressed high level of MHC molecules (H-2kd and I-A/I-E), CD11c and costimulatory molecules (CD40, CD80 and CD86). In contrast, the regulatory dendritic cells expressed moderate level of MHC molecules and extremely-low level of CD 11c and costimulatory molecules.

### Example 2: Comparison of cytokine expressed by Toll-like receptor ligand stimulation

The 2 x 10⁵ cells of dendritic cells prepared as described in Example 1 were incubated in the presence of 1 µg/mL of polyinosinic-polycytidylic acid (polyI:C) (Sigma Chemical Co., St. Louis, MO, US), 1 µg/mL of ultrapure LPS, 1 µg/mL of R848 (InvivoGen) or cytosine-phosphorothiolate-guanine oligonucleotide (CpG-ODN) (TCCATGACGTTCCTGATGCT: SEQ ID No. 5) in 200 µL of RPMI1640 medium (10 % fetal bovine serum (FCS), 20 mM of HEPES, 2-mercaptoethanol, penicillin and streptomycin in RPMI-1640 medium) in a round-bottom 96-well plate for 24 hours. The IL-10 and IL-6 in the supernatant were determined using a Cytometric Bead Array Kit (BD Biosciences Pharmingen) in accordance with the manufacturer's protocols.

The results are shown in Fig. 2. The stimulation of the regulatory dendritic cells by Toll-like receptor ligand resulted significantly lower level production of IL-6 and higher level production of IL-10 y the regulatory dendritic cells than the mature dendritic cells.

### Example 3: In vitro induction of Foxp3⁺CD4⁺CD25⁺ regulatory T cell by dendritic cells

It has been understood that Foxp3⁺CD4⁺CD25⁺ regulatory T cell plays a key role in modulation of various immune responses (J. D. Fontenot et al., Nat. Immunol., 6: 331-7 (2005); D.A. Vignali et al., Nat. Rev. Immunol., 8: 523-32 (2008); S. Hori et al., Science, 299: 1057-61 (2003)). It has been reported that regulatory dendritic cells and immunotolerogenic dendritic cells induce expression of Foxp3⁺CD4⁺CD25⁺ regulatory T cells and play a role in induction of tolerogenesis in mouse and human (S. Fujita et al., Blood, 110: 3793-803 (2007); M. Torisu et al., J. Gastroenterol., 43: 100-7 (2008); I. E. Dumitriu et al., J. Immunol., 182: 2795-807 (2009)). Therefore, it was examined whether the regulatory dendritic cells induce expression of Foxp3⁺CD4⁺CD25⁺ regulatory T cells or not.

### (1) Purification of CD4⁺CD25⁻ T cells

CD4⁺CD25⁻ T cells were isolated from spleen cells of BALB/c mouse with CD4⁺CD25⁺ regulatory T cell Isolation Kit and AutoMACS (Miltenyi Biotec GmbH) in accordance with the manufacturer's protocol. The purity of the isolated cells were confirmed to be 98 % or more by the FACS analysis using a peridinin-chlorophyll-protein (PerCP) labeled anti-CD4 monoclonal antibody (BD Pharmingen) and a phycoerythrin (PE) labeled anti-CD25 monoclonal antibody (Miltenyi Biotec GmbH).

### (2) In vitro induction of Foxp3⁺CD4⁺CD25⁺ T cells

5 x 10⁶ of CD4⁺CD25⁻ T cells isolated from BALB/c mouse was incubated together with 5 x 10⁵ of mature dendritic cells or regulatory dendritic cells isolated from BALB/c mouse in 1mL of RPMI medium using a 35 x 10 mm-type cell culture dish (Cornig Inc., Horseheads, NY, US) for 7 days. After the incubation, the cells were stained with PE labeled anti-Foxp3 monoclonal antibody (Clone FJK-16s, eBioscience, Inc., San Diego, CA, US), allophycocyanin (APC) labeled anti-CD25 monoclonal antibody (Clone PC61, BD Pharmingen) and PerPC labeled anti-CD4 monoclonal antibody (Clone RM4-5, BD Pharmingen) in accordance with the manufacturer's protocol. The fluorescence staining was examined by flow cytometry analysis using FloJo software.

### (3) Results

Results are shown in Figs. 3 and 4. Both of mature dendritic cells and regulatory dendritic cells produced the same number of CD4⁺CD25⁺ T cells. However, the Foxp3⁺CD4⁺CD25⁺ regulatory T cells were induced by regulatory dendritic cells, but were not induced by mature dendritic cells.

### Example 4: Treatment of colitis with CBA-pulsed regulatory dendritic cells

A therapeutic effect of the administration of regulatory dendritic cells on colitis was examined by following method:

### (1) Preparation of CBA

CBA was prepared according to the procedure which had been previously reported (Y. Cong et al., J. Exp. Med., 187: 855-64 (1998)). BALB/c mice were euthanized to extract its appendixes. Five appendixes were incised and added to 10 mL of phosphate buffered saline (PBS) containing 1.0 mm Silica-Sepharose (Lysing Matrix C, MP Biomedicals, Sorong, OH, US). After stirring for 5 minutes, the mixture was centrifuged at 5,000 g for 5 minutes at 4 °C to remove Silica-Sepharose and undissolved cells. Subsequently, the supernatant was centrifuged at 18,000 g for 30 minutes at 4 °C, and the lysate was sterilized with a syringe filter with pore size 0.2 µm. The protein concentration of the lysate was determined using a DC Protein Assay Kit (Bio-Rad Laboratories Inc., Hercules, CA, US) .

### (2) Preparation of CBA- or KLH-pulsed regulatory dendritic cell

The regulatory dendritic cells prepared as described in Example 1 were pulsed with either 50 µg/mL of CBA or 50 µg/mL of keyhole-limpet-hemocyanin (KLH) (Thermo Scientific, Rockford, IL, US) for 24 hours to induce CBA-pulsed dendritic cells (Reg-DCs_{CBA}) or KLH-pulsed dendritic cells (Reg-DCS_{KLH}), respectively.

### (3) Preparation of enteritis model and its treatment with dendritic cells

An enteritis model was prepared according to the procedure which had been previously reported (S. Kjellev et al., Int. Immunopharmacol., 6: 1341-54 (2006)). CD4⁺CD25⁻ T cells obtained from BALB/c mice were suspended in PBS to be 3 x 10⁵ cells/0.2 mL/mouse and then transplanted into a C.B-17 SCID mouse by the intraperitoneal administration. The day of transplantation was set as day 0. CD4⁺CD25⁻ T cell-nontransplanted mice were used as a control group (n=6). On day 0, with the CD4⁺CD25⁻ T cells, regulatory dendritic cells (n=7), Reg-DCs_{KLH} (n=6) or Reg-DCs_{CBA} (n=8), all of which had been obtained from the BALB/c mouse, were suspended in PBS to be 1 x 10⁶ cells/0.2 mL/mouse and were administered intraperitoneally. Mice which were administered with 0.2 mL PBS instead of the dendritic cells was used as a PBS group (n=10). The mice were weighed every week. The mice were euthanized 4 weeks after the cell transplantation to measure a length of colons.

### (4) Histological evaluation of colitis

The mice were euthanized 4 weeks after the cell transplantation and colons were taken from the mice. A transverse colon was extracted from the colon, fixed in 10 % neutral buffered formalin and embedded in paraffin. The tissue section of the colon was stained by H&E or a periodic acid-Schiff reagent (PAS). A degree of inflammation in the tissue section was determined according to the procedure which has been previously reported (S. Kjellev et al., Int. Immunopharmacol., 6: 1341-54 (2006)). The histological picture was scored according to the following criteria: 1) severity of inflammation: 0 none; 1 mild lymphocyte infiltration; 2 moderate lymphocyte infiltration or local crypt degeneration; 3 severe inflammation or plural crypt degeneration, and/or expression of erosion; 2) degree of inflammation: 0 none; 1 mucosal membrane; 2 submucosal layer; 3 transmural; 3) mucous amount: 0 normal; 1 slightly reduced; 2 moderately reduced or local absence; 3 severely reduced; 4 complete absence; and 4) degree of epithelial cell proliferation: 0 none; 1 mild increase in number of cells or crypt length; 2 moderate or locally significant increase; 3 significant increase - in entire portion of the tissue section. The histological score was determined as the sum of the above four individual parameters.

### (5) Results

The results are shown in Figs. 5 and 6. At 4 weeks after the cell transplantation, body weight of the mouse treated with Reg-DCs_{CBA} was significantly increased compared with that of the mouse administered with PBS (P < 0.01) (Fig. 5A). It was observed from macroscopic findings at 4 weeks after the cell transplantation that the colon of the mouse administered with PBS had thicker wall and shorter length. On the other hand, the colon of the mouse administered with the regulatory dendritic cells or the Reg-DCs_{CBA} was significantly longer than that of the mouse administered with PBS (P < 0.01) (Fig. 5B, 5C). From a histological appearance, colitis is characterized by severely excessive epithelial cell proliferation, mucus decrease, inflammatory cell infiltration, crypt degeneration, decrease of goblet cells and expression of erosion. In the C.B-17SCID mouse which was not administered with the dendritic cells, severely excessive epithelial cell proliferation, mucus decrease, inflammatory cell infiltration, crypt degeneration and decrease of goblet cells were observed and the mouse developed severe colitis. The similar histological changes in colon with the mouse which was administered with PBS was observed in the mouse which was administered with the antigen-nonpulsed regulatory dendritic cells or the Reg-DCs_{KLH}. In contrast, the treatment with the Reg-DCs_{CBA} resulted in mild histological changes, significantly decreased inflammatory cell infiltration and slightly decreased goblet cells (Fig. 5D) .

The histological score was determined on the basis of the severity of inflammation, the degree of inflammatory cell infiltration, the mucous amount and the degree of epithelial cell proliferation. The mouse which was administrated with Reg-DCs_{CBA} indicated significantly lower histological score than the mouse which was administered with PBS (Fig. 5E). The mouse treated with the regulatory dendritic cells showed the moderate weight loss and the slightly shortened colon, however the histological appearance further indicated the onset of moderate to severe colitis.

### Example 5: Expression of inflammatory cytokine in colon of enteritis model mouse

### (1) Measurement of expression of inflammatory cytokine mRNA

Each of four CD4⁺CD25⁻ T cells transplanted mice were administered with Reg-DCs_{CBA} or PBS as described in Example 4, and transverse colons were taken from said mice and were homogenized on a TissueLyser (QIAGEN K.K., Tokyo, Japan). All RNAs were extracted using RNeasy Plus MiniKit (QIAGEN K.K.). A complementary DNA (cDNA) was prepared from 10 µg of RNA using High Capacity cDNA Reverse Transcription Kit (Applied Biosystems, Inc., Foster City, CA, US). The mRNA expression of IL-10, IL-6 and IL-17A in the colon taken from the enteritis model mouse was measured with real time RT-PCR, and glyceraldehyde-3-phosphate dehydrogenase (GAPDH) expression was used as a control. A relative expression level was calculated according to the procedure which had been previously reported (Y. Tokumoto et al., J. Med. Virol., 79: 1120-7 (2007)).

### (2) Measurement of production quantity of inflammatory cytokine in ex vivo cultured colon

A 1 cm-tissue section was cut from the transverse colon, washed to remove feces, and washed with a sterile PBS three times. This section of the colon was added into the RPMI1640 medium (RPMI1640 medium containing 10 % FCS, 20 mM HEPES, 2-mercaptoethanol, penicillin and streptomycin) and incubated at 37 °C 5 % CO₂. The supernatant was collected 3 days after the start of incubation. Expressions of IL-17, IL-10, IL-6 tumor necrosis factor-α (TNF-α) and interferon-γ (IFN-γ) in the supernatant were measured with ELISA Kit (R&D Systems Company, Minneapolis, MN, U.S.A) and Cytometric Bead Array Kit (BD Biosciences Pharmingen).

Analysis of the cytokine mRNA expression revealed that in the colon of the mouse which was administered with Reg-DCs_{CBA}, both expression levels of IL-6 and IL-17A were decreased, and expression level of IL-10 was increased (Fig. 6A). Analysis of the supernatant of the colon culture showed that the production quantity of the inflammatory cytokines, IL-17 and TNF-α, in the colon of the mouse which was administered with Reg-DCs_{CBA} was significantly lower than that of the mouse which was administered with PBS (P < 0.05). However, there was no significant difference among the production quantities of IFN-γ, IL-6 and IL-10 (Fig. 6B) .

Example 6: Expression of transcription factor and inflammatory cytokine in mesenteric lymph nodes (MLN) cells of enteritis model mouse

In order to demonstrate the mechanism of enteritis suppression by Reg-DCs_{CBA}, transcription factor and cytokine in the mesenteric lymph nodes (MLN) cell were measured.

### (1) Measurement of mRNA expression of transcription factor

The CD4⁺CD25⁻ T cells transplanted mouse was administered with Reg-DCs_{CBA} or PBS as described in Example 4, and MLN cells were taken from said mouse and were homogenized on the TissueLyser (QIAGEN K.K.). All RNAs were extracted using RNeasy Plus MiniKit (QIAGEN K.K.). A complementary DNA (cDNA) was prepared from 10 µg of RNA using High Capacity cDNA Reverse Transcription Kit (Applied Biosystems, Inc.) The mRNA expression of Foxp3 and retinoic acid-based orphan receptor gamma t (RORγ T) in the MLN cells taken from the enteritis model mouse was measured with real time RT-PCR, and glyceraldehyde-3-phosphate dehydrogenase (GAPDH) expression was used as a control. A relative expression level was calculated according to the procedure which had been previously reported (Y. Tokumoto et al., J. Med. Virol., 79: 1120-7 (2007)).

### (2) Measurement of mRNA expression of inflammatory cytokine

The CD4⁺CD25⁻ T cells transplanted mouse was administered with Reg-DCs_{CBA} or PBS as described in Example 4, and MLN cells were taken from said mouse and were homogenized on the TissueLyser (QIAGEN K.K.). All RNAs were extracted using RNeasy Plus MiniKit (QIAGEN K.K.). A complementary DNA (cDNA) was prepared from 10 µg of RNA using High Capacity cDNA Reverse Transcription Kit (Applied Biosystems, Inc.). The mRNA expression of IL-17A, IL-6, IL-10 and TGF-β in the MLN cells taken from the colitis mouse model was measured with real time RT-PCR, and glyceraldehyde-3-phosphate dehydrogenase (GAPDH) expression as a control. A relative expression level was calculated according to the procedure which had been previously reported (Y. Tokumoto et al., J. Med. Virol., 79: 1120-7 (2007)).

### (3) Measurement of production quantity of inflammatory cytokine by ELISA

1 x 10⁶ MLN cells taken from the CD4⁺CD25⁻ T cells transplanted mouse which had been administered with Reg-DCs_{CBA} or PBS as described in Example 4, were incubated in the presence of 25 ng/mL of phorbol-12-myristic acid 13-acetate (PMA) (Sigma Chemical Co.) and 1 µg/mL of ionomycin (Sigma Chemical Co.) for 72 hours. The IL-6, interferon-y (IFN-γ), tumor necrosis factor α (TNF-α) and monocyte chemotactic protein-1 (MCP-1) in the supernatant were measured using the Cytometric Bead Array Kit (BD Biosciences Pharmingen) in accordance with the manufacturer's protocol. The IL-17 in the supernatant was measured using the ELISA kit (R&D Systems Company).

### (4) Results

The results are shown in Fig. 7. The Foxp3 expression level of the mouse which was administered with Reg-DCs_{CBA} was significantly higher than that of the mouse which was administered with PBS (P < 0.05) (Fig. 7A). The expression level of RORγ T mRNA of the mouse which was administered with Reg-DCs_{CBA} was significantly lower than that of the mouse which was administered with PBS (P < 0.05) (Fig. 7A). In addition, each mRNA expression level of IL-10 and TGF-β in the MLN from the mouse administered with Reg-DCs_{CBA} was significantly higher than that of the mouse administered with PBS (P < 0.05) (Fig. 7B). There was no significant difference in both expression levels of the IL-6 and IL-17A in the MLN cells between the mouse which was administered with Reg-DCs_{CBA} and the mouse which was administered with PBS (Fig. 7B). However, the production of IL-6 in MLN cell of the mouse administered with Reg-DCs_{CBA} was significantly lower than that of the mouse administered with PBS (Fig. 7C). Further, the administration of Reg-DCs_{CBA} resulted in significantly decreased production of IFN-γ, TNF-α and MCP-1 in MLN of the enteritis model mouse (P < 0.01) (Fig. 7C).

### Example 7: In vivo induction of Foxp3⁺CD4⁺CD25⁺ regulatory T cells and IL-10-producing CD4⁺CD25⁺ T cells by Reg-DCs_{CBA}

In order to examine induction of Foxp3⁺CD4⁺CD25⁺ regulatory T cells and of IL-10-producing CD4⁺CD25⁺ T cells from Foxp3⁻CD4⁺CD25⁻ T cells by the Reg-DCs_{CB} in the periphery in vivo, each number of induced Foxp3⁺CD4⁺CD25⁺ regulatory T cells and induced IL-10-producing CD4⁺CD25⁺ T cells was counted according to the following procedure.

### (1) Preparation of mouse and MLN

The CD4⁺CD25⁻ T cells transplanted mouse was administered with Reg-DCs_{CBA} or PBS as described in Example 4, and MLN cells were taken from said mouse at 1, 3 and 5 week(s) after the CD4⁺CD25⁻ T cells transplantation and then incubated in 200 µL of RPMI1640 medium in the presence of 25 ng/mL of PMA (Sigma Chemical Co.) and 1 µg/mL of ionomycin (Sigma Chemical Co.) for 72 hours.

### (2) Measurement of Foxp3⁺CD4⁺CD25⁺ regulatory T cells

To examine a proportion of Foxp3⁺CD4⁺CD25⁺ regulatory T cells in the MLN, the MLN was taken at 1, 3 and 5 week(s) after the CD4⁺CD25⁻ T cells transplantation and incubated in 200 µL of RPMI1640 medium in the presence of 25 ng/mL of PMA (Sigma Chemical Co.) and 1 µg/mL of ionomycin (Sigma Chemical Co.) in a round-bottom 96-well plate for 72 hours. Then, the cultured cells were stained with PE labeled anti-Foxp3 monoclonal antibody, APC labeled anti-CD25 monoclonal antibody and PerCP labeled anti-CD4 monoclonal antibody in accordance with the recommended protocol by the Manufacturer. The fluorescence staining was examined by flow cytometry analysis.

### (3) Measurement of IL-10-producing CD4⁺CD25⁺ regulatory T cells

To examine a proportion of IL-10-producing CD4⁺CD25⁺ regulatory T cells in the MLN, the MLN was taken at 2, 4, 7 and 14 days after CD4⁺CD25⁻ T cells transplantation and incubated in 200 µL of RPMI1640 medium in the presence of 25 ng/mL of PMA (Sigma Chemical Co.) and 1 µg/mL of ionomycin (Sigma Chemical Co.) in a round-bottom 96-well plate for 24 hours. For the last 3 hours incubation, GolgiStop (BD Pharmingen) was added thereto. Then, the cells were incubated with PerCP labeled anti-CD4 monoclonal antibody and APC labeled anti-CD25 monoclonal antibody in a dark room at room temperature for 15 minutes, and then, fixed and permeabilized with FIX & PERM Kit (Caltag Laboratories Inc., Burlingame, CA, US). The resulting cells were stained with PE labeled rat anti-mouse IL-10 monoclonal antibody (Clone JES5-16E3, D Pharmingen) at room temperature for 20 minutes. The fluorescence staining was examined by flow cytometry analysis.

### (4) Results

The results are shown in Fig. 8. The results by the flow cytometry analysis showed that on 7 days after CD4⁺CD25⁻ T cells transplantation, both of proportions of the Foxp3⁺CD4⁺CD25⁺ T cells and the IL-10-producing CD4⁺CD25⁺ regulatory T cells in the MLN of the mouse administered with Reg-DCs_{CBA} were significantly increased compared with that of the mouse administered with PBS.

### Example 8: Proteome analysis of CBA

### (1) Preparation of CBA

CBA was prepared as described in Example 4.

### (2) 2D Gel electrophoresis and imaging

Prior to the isoelectric focusing electrophoresis, the CBA sample was treated with 2-D Clean-Up Kit (GE Healthcare Bioscience Co., Ltd., Piscataway, NJ, US). The resulting pellet was resuspended into buffered solution (30 mM of Tris-HCl, pH 8.5, containing 7 M urea, 2 M thiourea, 4 % CHAPS and PlusOne Protease Inhibitor Mix). The sample was dissolved into swelling buffer solution (7 M urea, 2 M thiourea, 4 % CHAPS, 0.5 % IPG buffer, pH 3 to 10, and 1 % DTT) and was swollen with the 24 cm of Immobiline DryStrip, pH 3 to 10 (GE Healthcare Bioscience Co., Ltd.) at 20 °C for 10 hours. The isoelectric focusing electrophoresis was carried out with IPGphor II at 20 °C for a total of 45 kVh. Next, the resulting IPG gel was equilibrated with 50 mM Tris-HCl, pH 8.8, containing 6 M urea, 30 % glycerol, 2 % SDS, 0.002 % of bromophenol blue and 0.5 % DTT for 15 minutes. And then, it was alkylated in buffer solution containing 50 mM of Tris-HCl, pH 8.8, 6 M urea, 30 % glycerol, 2 % SDS, 0.002 % bromophenol blue and 4.5 % iodoacetamide for 15 minutes. Soon after that, the resulting strip was applied onto the 12.5 % SDS-PAGE gel and electrophoresed with EttanDALTsix electrophoresis system (GE Healthcare Bioscience Co., Ltd.) under a condition of 2 W at 30 °C for 16 hours. The gel was stained with Deep Purple Total Protein Stain (GE Healthcare Bioscience Co., Ltd.) according to the standard protocol. The stained gel was scanned with Ettan DIGE Imager (GE Healthcare Bioscience Co., Ltd.), and the resulting image was analyzed with ImageMaster 2D Platinum (GE Healthcare Bioscience Co., Ltd.). Each spot was quantified based on its relative volume (a spot volume divided by the total volume of all gel spots).

### (3) Mass spectrometry

A protein spot was cut from the gel, washed, and digested by pig trypsin protease in the gel. The trypsin peptide was extracted by sonication. Using nano LC-AccuSpot (Shimadzu Corporation, Kyoto, Japan), HPLC and MALDI samples were prepared and spotted onto µ FOCUS MALDI plate (Shimadzu Corporation). The tandem TOF/TOF mass spectrometry was performed with Axima-TOF² mass spectrometer (Shimadzu Corporation). The protein was identified by "MASCOT" MS/MS ion search engine (Matrix Science, London, UK) and the protein database provided by National Center for Biotechnology Information.

### (4) Results

The results are shown in Fig. 9. All of three individual experiments gave the same results. The proteome analysis from the 2D DIGE imaging showed 14 main spots on the spot map (Fig. 9A). These spots were cut from the gel by mass spectrometry. The results of inquiry with the spots to the database are shown in Table 1. From the proteome analysis, the mouse CA I was identified as the primary protein antigen of CBA.
[Table 1]

**Table.1 Identification of proteins from mouse cecal bacterial antigen**

| Spot No. | %Volume | Experimental mol. mass (kDa)/pl | Protein Name | Theoretical mol. mass (kDa)/ pl | MOWSE Score | Queries matched | Sequence coverage |
|---|---|---|---|---|---|---|---|
| 1 | 3.347 | 30.0 / 7.00 | Carbonic anhydrase 1 [Mus musculus) | 28.4 / 6.44 | 1003 | 20 | 48% |
| 2 | 3.309 | 73.0 / 5.88 | Serum albumin precursor [Mus musculus] | 70.8 / 5.75 | 904 | 16 | 22% |
| 3 | 1.547 | 72.0 / 5.78 | Serum albumin precursor [Mus musculus] | 70.8 / 5.75 | 827 | 15 | 18% |
| 4 | 0.984 | 30.0 / 4.07 | Carbonic anhydrase 1 [Mus musculus] | 28.4 / 6.44 | 673 | 14 | 47% |
| 5 | 0.962 | 30.0 / 6.82 | Carbonic anhydrase 1 [Mus musculus] | 28.4 / 6.44 | 973 | 15 | 57% |
| 6 | 0.935 | 53.0 / 5.92 | Selenium binding protein 1 [Mus musculus] | 53.1 / 5.87 | 1388 | 21 | 43% |
| 7 | 0.903 | 73.0 / 5.71 | Serum albumin precursor [Mus musculus] | 70.8 / 5.75 | 1071 | 19 | 22% |
| 8 | 0.899 | 43.0 / 4.32 | Heat shock protein 70 cognate [Mus musculus] | 71.1 / 5.37 | 1261 | 21 | 32% |
| | | | Serine proteinase inhibitor, clade A, member In [Mus musculus] | 46.2 / 5.44 | 105 | 2 | 7% |
| 9 | 0.892 | 30.0 / 4.35 | Carbonic anhydrase 1 [Mus musculus] | 28.4 / 6.44 | 412 | 9 | 39% |
| | | | Carbonic anhydrase 3 [Mus musculus] | 29.7 / 6.89 | 137 | 3 | 18% |
| 10 | 0.849 | 30.0 / 4.24 | Carbonic anhydrase 1 [Mus musculus] | 28.4 / 6.44 | 438 | 6 | 40% |
| | | | Carbonic anhydrase 3 [Mus musculus] | 29.7 / 6.89 | 101 | 3 | 12% |
| 11 | 0.835 | 62.0 / 4.96 | Chaperonin GroEL [Dorca longientena DSM 13814] | 57.5 / 4.86 | 321 | 5 | 8% |
| | | | Chaperonin GroEL [Enbacterium ventriosum ATCC 27560] | 57.1 / 4.77 | 317 | 5 | 9% |
| | | | Chaperonin GroEL [Ruminococcus torques ATCC 27756] | 57.6 / 4.81 | 186 | 3 | 5% |
| 12 | 0.617 | 31.0 / 4.60 | Not identified | | | | |
| 13 | 0.615 | 31.0 / 5.36 | Elastase 3B, pancreatic [Mus musculus] | 29.7 / 5.39 | 239 | 5 | 17% |
| 14 | 0.609 | 54.0 / 6.04 | Selenium binding protein 1 [Mus musculus] | 53.1 / 5.87 | 1306 | 23 | 45% |

### Example 9: CA I expression in colon

Colons taken from SCID mouse which was not administered with CD4⁺CD25⁻ T cells, SCID mouse with enteritis induced by the CD4⁺CD25⁻ T cells as described in Example 4, and an SCID mouse which was administered with both of the CD4⁺CD25⁻ T cells and Reg-DCs_{CBA} were examined to determine CA I expression by immunohistochemical staining.

### (1) Immunohistochemical staining

A 5 µm frozen tissue section of the colon was fixed with acetone. The section was treated with method containing 1 % hydrogen peroxide for 20 minutes and then with PBS containing 0.1 % Tween-20 for 10 minutes to inactivate endogenous peroxidase activity. The tissue section was inactivated with endogenous avidin/biotin blocking kit (Nichirei Corporation, Tokyo, Japan) pretreated with Rabbit Serum (Nichirei Corporation), followed by incubation in the presence of 1:50 diluted biotinylated goat anti-human CA I antibody (Rockland Immunochemicals Inc., Philadelphia, PA, US) at 4 °C overnight. Then, the tissue section was treated with horseradish-conjugated streptavidin (Nichirei Corporation), followed by incubation with Simple Stain DAB solution (Nichirei Corporation). Finally, the tissue section was counterstained with hematoxylin and dried to prepare a sample.

### (2) Results

The results are shown in Fig. 10. The colon of SCID mouse which was administered with only CD4⁺CD25⁻ T cells expressed significantly decreased amount of CA I compared with both of the SCID mouse which was not administered with CD4⁺CD25⁻ T cells and the SCID mouse which was administered with both of CD4⁺CD25⁻ T cells and Reg-DCs_{CBA}.

### Example 10: Therapeutic effect on colitis by CA I-pulsed regulatory dendritic cells

To examine whether the CA I-pulsed regulatory dendritic cells improves enteritis of the CD4⁺CD25⁻ T cell-transplanted inflammatory bowel disease model mouse, CA I-pulsed regulatory dendritic cells were administered to the mouse which was administered with CD4⁺CD25⁻ T cells and the effect was evaluated.

### (1) Preparation of mouse CA I

The mouse CA I was prepared by cell free protein synthesis system using wheat germ ribosomal RNA (K. Madin et al., Proc. Natl. Acad. Sci. USA, 97: 559-64 (2000)), which have no risk of contamination of LPS. The expression plasmid (pEU-mCA1-His) was prepared as follows. The mouse CA1 gene was amplified with each 0.3 µM or less of primers mCA1F and mCA1R by using KOD-Plus-Ver. 2 kit (Toyobo Co., Ltd., Osaka, Japan).
mCA1F: 5'-AATAAGATATCATGGCAAGTGCAGACTGG-3' (SEQ ID No. 6)
mCA1R: 5'-TGCTGGACTAGTAAATGAGGCTCTGACTGTTC-3' (SEQ ID No. 7)

The CA I was synthesized with Robotic Protein Synthesizer (trademark) DT (Cellfree Science Co., Ltd., Matsuyama, Japan) in accordance with the manufacturer's protocol as previously reported (T. Sawasaki et al., FEBS Lett., 514: 102-5 (2002)). The resulting His-fused CA I was purified with Ni Sepharose High Performance (GE Healthcare Bioscience Co., Ltd.). An AcTEV (trademark) protease (Invitrogen Corporation) was used to remove a His tag from the fusion protein and then the solution was dialyzed against PBS using Mini Dialysis Kit (GE Healthcare Bioscience Co., Ltd.).

Proteins prepared using the empty plasmid non-genetically recombined with CA I gene sequences by the cell free protein synthesis system in accordance with Preparation of CA I were used as CA I-control.

### (2) Preparation and pulsing of regulatory dendritic cell

The regulatory dendritic cells were prepared according to the procedures described in Example 1. The prepared regulatory dendritic cells were pulsed with 6 µg/mL of CA I or CA I-control for 24 hours to produce CA I-pulsed regulatory dendritic cells (Reg-DCs_{CA1}) or CA I-control-pulsed regulatory dendritic cells (Reg-DCs_{CA1-control}), respectively.

### (3) Preparation of enteritis model mouse and treatment with dendritic cell

The enteritis model mouse was prepared according to the procedures described in Example 4. 3 x 10⁵ cells/mouse of the CD4⁺CD25⁻ T cells taken from BALB/c mouse were administered intraperitoneally and transplanted to a B-17 SCID mouse. The transplanting date was set as day 0. The C.B-17 mice which was not administered with CD4⁺CD25⁻ T cells were used as a control group (n=5). On day 0, together with the CD4⁺CD25⁻ T cells, each of Reg-DCs_{CBA} (n=7), Reg-DCs_{CA1-control} (n=8) and Reg-DCs_{CA1} (n=10) taken from the BALB/c mouse was administered intraperitoneally in of 1 x 10⁶ cells/mouse. The mice which were administered with PBS instead of the dendritic cells was defined as a PBS group (n=13). The mice were weighed every week. The mice were euthanized 4 weeks after the cell transplantation to determine a length of colons.

### (4) Histological evaluation of colitis

The mouse was euthanized 4 weeks after the cell transplantation to take a colon from it. A transverse colon was extracted, fixed in 10 % neutral buffered formalin and embedded in paraffin. The tissue section was stained by H&E or PAS. A degree of inflammation in the tissue section was rated according to the procedure described in Example 4.

### (5) Measurement of mRNA expression of inflammatory cytokine in colon

Each mRNA expression of IL-17, IL-10 and TGF-β in the colon was measured according to the procedure described in Example 5.

### (6) mRNA Expression of Transcription factor and inflammatory cytokine in MLN

Each mRNA expression of IL-17, IL-10, TGF-β, Foxp3 and RORγ T in the MLN was measured according to the procedure described in Example 6.

### (7) Measurement of production quantity of inflammatory cytokine in ex vivo cultured colon

The IL-6, IL-17, TNF-α and IFN-γ in the colon culture supernatant were measured according to the procedure described in Example 5.

### (8) Measurement of production quantity of inflammatory cytokine in MLN cell

The IL-6, IL-17, TNF-α, IFN-γ and MCP-1 in the culture supernatant of MLN cell were measured according to the procedure described in Example 6.

### (9) Results

The results were shown in Fig. 11. At four weeks after the cell transplantation, the body weight of the mouse administered with Reg-DCs_{CA1} was significantly increased compared with the mouse administered with PBS (P=0.006) (Fig. 11A). The macroscopic findings at 4 weeks after the cell transplantation showed that the mouse administered with Reg-DCs_{CA1} had significantly longer colon than the mouse administered with PBS (P < 0.0001) (Fig. 11B). The mouse administered with Reg-DCs_{CA1} indicated significantly lower histological score than the mouse administered with PBS (Fig. 11C).

The measurements of mRNA expression showed that in the colon and the MLN of the mouse administered with Reg-DCs_{CA1}, IL-17A expression was decreased, whereas both expressions of IL-10 and TGF-β were increased (P < 0.05) (Figs. 11D and 11E). The Foxp3 expression in the MLN of the mouse administered with Reg-DCs_{CA1} was significantly increased compared with the mouse administered with PBS (P < 0.05) (Fig. 11E). The expression of RORγ T mRNA in the mouse administered with Reg-DCs_{CA1} was decreased compared with the mouse administered with PBS (P < 0.05) (Fig. 11E).

The analysis of the cultured supernatant of the colon showed that each production quantity of IL-17, IFN-γ and TNF-α in the colon of the mouse administered with Reg-DCs_{CA1} was significantly smaller than that of the mouse administered with PBS (P < 0.05) (Fig. 11F). Furthermore, the administration of Reg-DCs_{CA1} decreased production quantity of IL-6, IL-17, TNF-α, IFN-γ and MCP-1 in the MLN cell of the enteritis model mouse significantly (P < 0.01) (Fig. 11G).

### Example 11

### (1) Preparation of mouse CA I

cDNA of Mouse CA I (hereinafter referred to as mCA1) was amplified using the following primers:
Forward: ATGGCAAGTGCAGACTGGGGA (SEQ ID No. 8);
Reverse: CCTTGCGGATCCTCAAAATGAGGCTCTGACTG (SEQ ID No. 9).

The amplified DNA was inserted into a plasmid pET 45b (Merk kGaA, Darmstadt, Germany) at PshAI and BamHI sites to assemble a pET-mCA1-His6 construct expressing mCA1 tagged with 6 His at the N-terminal. The construct was confirmed by the cycle sequencing method. The His-CA1 was expressed in Escherichia coli BL21 (DE3), purified with the metal (Ni2+) affinity chromatography and then dialyzed in PBS to remove imidazole. It was further treated with EndTrap (trademark) red column (Hyglos GmbH, Regensburg, Germany) to remove endotoxin. Following SDS-PAGE, the gel was stained with Deep Purple Total Protein Stain (GE Healthcare). The gel image was analyzed by an ImageQuant TL (GE Healthcare) to calculate the purity of the protein. Control proteins prepared using the empty plasmid pET 45b in the same way as the mCA1 was used as a CAI-control.

### (2) Antigen and administration regimen

In order to evaluate improvement of enteritis by oral administration of mCA1 to the CD4⁺CD25⁻ T cell-transplanted enteritis model mouse, the C.B-17 SCID mouse was continuously fed solution of 0.6 % mCA1 as drinking water for 5 days (from the day -7 to the day -2) according to the previous report (A. M. Faria et al., J. Autoimmun., 20: 135-45 (2003)). The mice were individually contained in cages and consumed 4.5 ± 0.5 mL/day of the solution of mCA1, and an average consumption per group was 5.0 ± 0.5 mL/day. Total dose of mCA1 per day was calculated based on the average consumption (5 mL/day). A feeding bottle containing the solution of mCA1 in PBS was changed twice a day to avoid contamination. This continuous feeding was continued for 5 days. To the control group, either PBS or CA I-control was administrated for 5 days. Seven days after the oral administration of mCA1 (day 0), CD4⁺CD25⁻ T cells (3 x 10⁵ cells/mouse) were intraperitoneally administered into the SCID mice.

### (3) Evaluation

Measurement of body weight and colon length of the mice, histological evaluation of colitis, measurement of mRNA expression of inflammatory cytokine in colon, mRNA expression of transcription factor and inflammatory cytokine in MLN cells, measurement of production quantity of inflammatory cytokine in the ex vivo cultured colon, and measurement of production quantity of inflammatory cytokine in MLN cells were carried out as described in Example 10.

### (4) Results

The oral administration of CA I improved enteritis of the CD4⁺CD25⁻ T cell-transplanted inflammatory bowel disease model mouse. At 4 weeks after the cell transplantation, body weight of the mouse administered with mCA1 was significantly increased compared with the mouse administered with PBS (P < 0.05) (Fig. 12A). At four weeks after the cell transplantation, it was observed macroscopically that the mouse administered with mCA1 had significantly longer colon than the mouse administered with PBS (P < 0.001) (Fig. 12B). The mouse administered with mCA1 indicated significantly lower histological score than the mouse administered with PBS (P < 0.05) (Fig. 12C).

The inflammatory bowel disease model mouse administered with mCA1 expressed significantly increased level of mRNA of IL-10 in colon compared with the mouse administered with PBS (P < 0.05) (Fig. 12D). Expressions of IL-17 and RORγ T mRNA in MLN cells were significantly decreased in the mouse administered with mCA1 compared with the mouse administered with PBS (P < 0.05) (Fig. 12E).

The measurement results of the cytokine production in the tissue section of the colon and in the MLN cells taken from the inflammatory bowel disease model mice which were administered with PBS or mCA1 showed that the mouse administered with mCA1 produced significantly decreased quantities of IL-6 and MCP-1 in colon compared with the mouse administered with PBS (P < 0.05) (Fig. 12F). In addition, the oral administration of mCA1 significantly decreased production of IL-6, IL-17, MCP-1 and TNFα, and increased production of IFNγ (P < 0.05) (Fig. 12G).

These results suggest that CBA-pulsed regulatory dendritic cell inhibits progress of enteritis of CD4⁺CD25⁻ T cells-transplanted enteritis model mouse; CA I, a primary antigen of CBA, plays an important role in suppression of development of colitis by the regulatory dendritic cells; the CA I-pulsed regulatory dendritic cells induces the Foxp3⁺ regulatory T cells in MLN and to decreases T-helper-17 cells; and the CBA-pulsed regulatory dendritic cells induces the Foxp3⁺CD4⁺CD25⁺ T cells and IL-10-producing CD4⁺CD25⁺ T cells in vivo. The results of the present experiments also show that the treatment with Reg-DCs_{CBA} improves clinical and histopathological severity of enteritis. Further, the results show that only CBA pulsed regulatory dendritic cells suppresses enteritis, but not KLH pulsed regulatory dendritic cells. Furthermore, a primary protein of CBA is identified as CA I. Therefore, from these findings it has been showed that the regulatory dendritic cells antigen-specifically inhibits enteritis, and that both of CBA and its primary antigen CA I can be the principal targets for inflammatory bowel disease. In this experiment, the significantly decreased CA I expression in the CD4⁺CD25⁻ T cell-transplanted enteritis was maintained by administration of Reg-DCs_{CBA}. Also, Reg-DCs_{CA1} has been shown to have depressant effect on enteritis in the enteritis model mouse. Therefore, it has been shown that CA I is the antigen specific to the inflammatory bowel disease. In addition, the oral administration of CA I exerts depressant effect on enteritis in inflammatory bowel disease model mouse. This result also supports that CA I is the antigen specific to the inflammatory bowel disease.

Accordingly, from these results, it has been revealed that CA I-pulsed regulatory dendritic cells control the balance between Foxp3⁺CD4⁺CD25⁺ T cells and Th17 cells and thus inhibit the progress of CD4⁺CD25⁻ T cells-transplanted enteritis. This suggests that the cell therapy by the CA I-pulsed regulatory dendritic cells can be a novel method for treating inflammatory bowel disease. Further, this also suggests that CA I can be a therapeutic agent or a preventive agent for inflammatory bowel disease.

## Claims

1. Carbonic anhydrase I, a carbonic anhydrase I-specific tolerogenic antigen presentation cell or a carbonic anhydrase I-specific regulatory T cell for use in a method of inducing immune tolerance and thereby treating or preventing inflammatory bowel disease.

2. Carbonic anhydrase I for use according to claim 1, wherein the method is cell therapy based on a tolerogenic antigen presentation cell.

3. The carbonic anhydrase I-specific tolerogenic antigen presentation cell for use according to claim 1, or carbonic anhydrase I for use according to claim 2, wherein the tolerogenic antigen presentation cell is a regulatory dendritic cell.

4. Carbonic anhydrase I for use according to claim 1, wherein the method comprises pulsing a tolerogenic antigen presentation cell *ex vivo* with carbonic anhydrase I.

5. Carbonic anhydrase I for use according to claim 4, wherein the method comprises:
- taking the tolerogenic antigen presentation cell from a patient;
- pulsing the tolerogenic antigen presentation cell with carbonic anhydrase I; and
- administering the pulsed tolerogenic antigen presentation cell into the patient.

6. Carbonic anhydrase I for use according to claim 4 or 5, wherein the tolerogenic antigen presentation cell is a regulatory dendritic cell.

7. An *ex vivo* method for producing an antigen-specific tolerogenic antigen presentation cell, comprising pulsing a tolerogenic antigen presentation cell obtained from a patient with carbonic anhydrase I.

8. An *ex vivo* method for producing an antigen-specific regulatory T cell, comprising:
- preparing a tolerogenic antigen presentation cell and a naive T cell;
- pulsing the tolerogenic antigen presentation cell with carbonic anhydrase I; and
- contacting the pulsed tolerogenic antigen presentation cell with the naive T cell.

9. The method of claim 7 or 8, wherein the tolerogenic antigen presentation cell is a regulatory dendritic cell.

10. An isolated carbonic anhydrase I-specific immunogenic antigen presentation cell.

11. The cell of claim 10, wherein the immunogenic antigen presentation cell is a regulatory dendritic cell.

12. An isolated carbonic anhydrase I-specific regulatory T cell.

## Patentansprüche

1. Carboanhydrase I, eine Carboanhydrase I-spezifische tolerogene Antigen-Präsentationszelle oder eine Carboanhydrase I-spezifische regulatorische T-Zelle zur Verwendung in einem Verfahren zum Induzieren von Immuntoleranz und dadurch Behandeln oder Verhüten entzündlicher Darmerkrankung.

2. Carboanhydrase I zur Verwendung nach Anspruch 1, wobei das Verfahren eine Zelltherapie beruhend auf einer tolerogenen Antigen-Präsentationszelle ist.

3. Carboanhydrase I-spezifische tolerogene Antigen-Präsentationszelle zur Verwendung nach Anspruch 1, oder Carboanhydrase I zur Verwendung nach Anspruch 2, wobei die tolerogene Antigen-Präsentationszelle eine regulatorische dendritische Zelle ist.

4. Carboanhydrase I zur Verwendung nach Anspruch 1, wobei das Verfahren ein Pulsen einer tolerogenen Antigen-Präsentationszelle *ex vivo* mit Carboanhydrase I umfasst.

5. Carboanhydrase I zur Verwendung nach Anspruch 4, wobei das Verfahren umfasst:
Entnehmen der tolerogenen Antigen-Präsentationszelle von einem Patienten;
Pulsen der tolerogenen Antigen-Präsentationszelle mit Carboanhydrase I; und
Verabreichen der gepulsten tolerogenen Antigen-Präsentationszelle in den Patienten.

6. Carboanhydrase I zur Verwendung nach Anspruch 4 oder 5, wobei die tolerogene Antigen-Präsentationszelle eine regulatorische dendritische Zelle ist.

7. *Ex*-*vivo*-Verfahren zum Herstellen einer Antigen-spezifischen tolerogenen Antigen-Präsentationszelle, umfassend ein Pulsen einer tolerogenen Antigen-Präsentationszelle, die von einem Patienten erlangt wurde, mit Carboanhydrase I.

8. *Ex*-*vivo*-Verfahren zum Herstellen einer Antigen-spezifischen regulatorischen T-Zelle, umfassend:
Zubereiten einer tolerogenen Antigen-Präsentationszelle und einer naiven T-Zelle;
Pulsen der tolerogenen Antigen-Präsentationszelle mit Carboanhydrase I; und
Inkontaktbringen der gepulsten tolerogenen Antigen-Präsentationszelle mit der naiven T-Zelle.

9. Verfahren nach Anspruch 7 oder 8, wobei die tolerogene Antigen-Präsentationszelle eine regulatorische dendritische Zelle ist.

10. Isolierte Carboanhydrase I-spezifische immunogene Antigen-Präsentationszelle.

11. Zelle nach Anspruch 10, wobei die immunogene Antigen-Präsentationszelle eine regulatorische dendritische Zelle ist.

12. Isolierte Carboanhydrase I-spezifische regulatorische T-Zelle.

## Revendications

1. Anhydrase carbonique I, cellule de présentation d'antigène tolérogénique spécifique d'anhydrase carbonique I ou cellule T régulatrice spécifique d'anhydrase carbonique I pour l'utilisation dans un procédé de déclenchement de tolérance immunitaire et ainsi de traitement ou de prévention de maladie abdominale inflammatoire.

2. Anhydrase carbonique I pour l'utilisation selon la revendication 1, dans laquelle le procédé est une thérapie cellulaire fondée sur une cellule de présentation d'antigène tolérogénique.

3. Cellule de présentation d'antigène tolérogénique spécifique à l'anhydrase carbonique I pour l'utilisation selon la revendication 1, ou anhydrase carbonique I pour l'utilisation selon la revendication 2, dans laquelle la cellule de présentation d'antigène tolérogénique est une cellule dendritique régulatrice.

4. Anhydrase carbonique I pour l'utilisation selon la revendication 1, dans laquelle le procédé comprend la pulsation d'une cellule de présentation d'antigène tolérogénique *ex vivo* avec anhydrase carbonique I.

5. Anhydrase carbonique I pour l'utilisation selon la revendication 4, dans laquelle le procédé comprend :
- le prélèvement de la cellule de présentation d'antigène tolérogénique à partir d'un patient ;
- la pulsation de la cellule de présentation d'antigène tolérogénique avec anhydrase carbonique I ; et
- l'administration de la cellule de présentation d'antigène tolérogénique pulsée dans le patient.

6. Anhydrase carbonique I pour l'utilisation selon la revendication 4 ou 5, dans laquelle la cellule de présentation d'antigène tolérogénique est une cellule dendritique régulatrice.

7. Procédé *ex vivo* pour produire une cellule de présentation d'antigène tolérogénique spécifique d'antigène, comprenant la pulsation d'une cellule de présentation d'antigène tolérogénique obtenue d'un patient avec anhydrase carbonique I.

8. Procédé *ex vivo* pour produire une cellule T régulatrice spécifique d'antigène, comprenant :
- la préparation d'une cellule de présentation d'antigène tolérogénique et d'une cellule T naïve ;
- la pulsation de la cellule de présentation d'antigène tolérogénique avec anhydrase carbonique I ; et
- la mise en contact de la cellule de présentation d'antigène tolérogénique pulsée avec la cellule T naïve.

9. Procédé selon la revendication 7 ou 8, dans laquelle la cellule de présentation d'antigène tolérogénique est une cellule dendritique régulatrice.

10. Cellule de présentation antigène immunogénique spécifique d'anhydrase carbonique I isolée.

11. Cellule selon la revendication 10, dans laquelle la cellule de présentation antigène immunogénique est une cellule dendritique régulatrice.

12. Cellule T régulatrice spécifique d'anhydrase carbonique I isolée.
